(19) Europäisches Patentamt
European Patent Office
Office européen des brevets

(11) EP 2 887 413 A1

(12) **EUROPEAN PATENT APPLICATION**

(43) Date of publication:
**24.06.2015 Bulletin 2015/26**

(51) Int Cl.:
***H01L 51/30*** *(2006.01)* ***C07D 241/02*** *(2006.01)*
***C08G 61/12*** *(2006.01)*

(21) Application number: **13198931.1**

(22) Date of filing: **20.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Solvay SA**
**1120 Bruxelles (BE)**

(72) Inventors:
- **Caille, Jean Raphael**
  **5000 Namur (BE)**
- **Lorent, Karol**
  **7110 Strepy-Bracquegnies (BE)**
- **Fenoll, Mathieu**
  **1030 Schaerbeek (BE)**

(74) Representative: **Dr. Langfinger & Partner**
**In der Halde 24**
**67480 Edenkoben (DE)**

(54) **Use of compounds comprising a piperazine structural element in organic electronic devices and compounds and polymers comprising a piperazine structural element**

(57) Use of compounds comprising at least one of structural elements (1) to (5) or of oligomers or polymers comprising units obtained from compounds of formulas (1) to (5

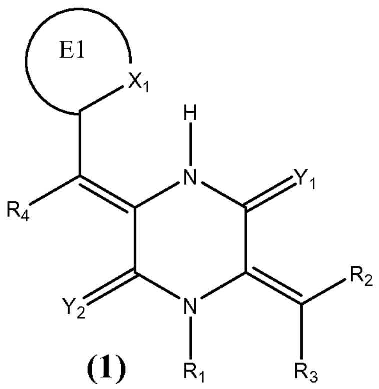

**(1)**

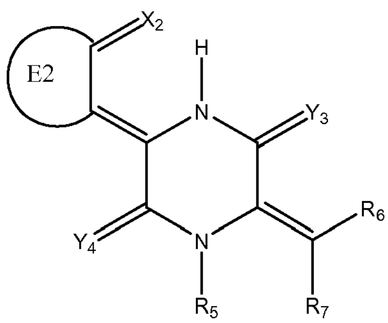

**(2)**

EP 2 887 413 A1

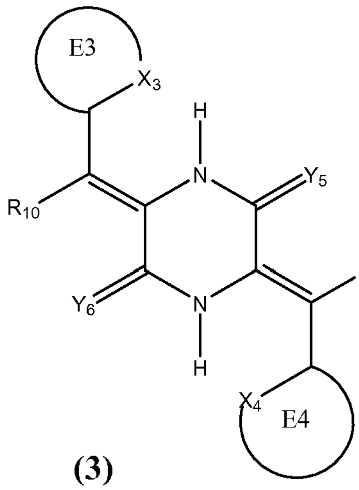

(3)

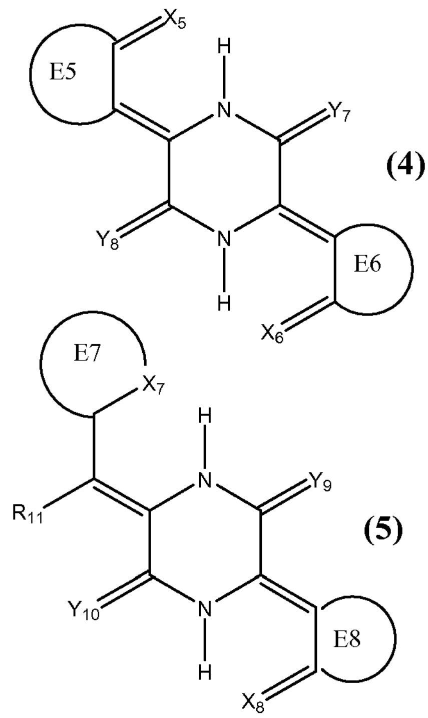

(4)

(5)

as organic semiconductors in organic electronic devices.

**Figure 1**

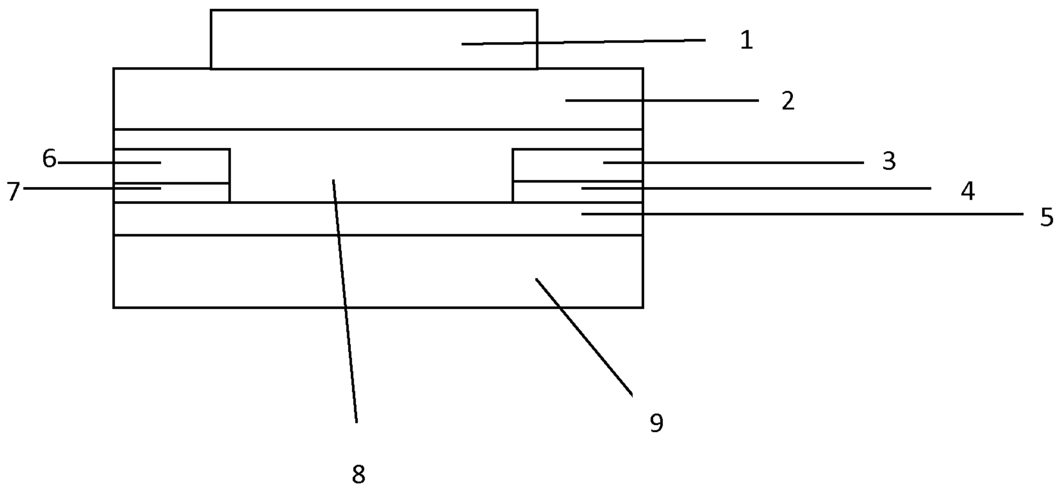

## Description

The present invention relates to the use of compounds comprising a 2,5-diketopiperazine structural element and capable of forming intramolecular hydrogen bonds in organic electronic devices and to novel compounds comprising a diketopiperazine structural element and intramolecular hydrogen bonds as such.

Organic semiconductors are currently under intensive investigation and research as materials which may be used in organic electronic devices, such as transistors, OLEDs, RFID tags, display backplanes (e.g. for e-readers or similar devices), organic photovoltaic devices or the like.

Compounds comprising 2,5-diketopiperazine structural elements and forming intramolecular hydrogen bonds have been described in the literature for a number of applications.

Gompper et al., Angew. Chem. Int. Ed. 1992, 31, No. 9, 1202ff disclose indigoid para-quinodimethanes for use in coloration, especially textile dyeing.

In the document, compounds 21a), 21b) depicted below are shown on page 1204, right column

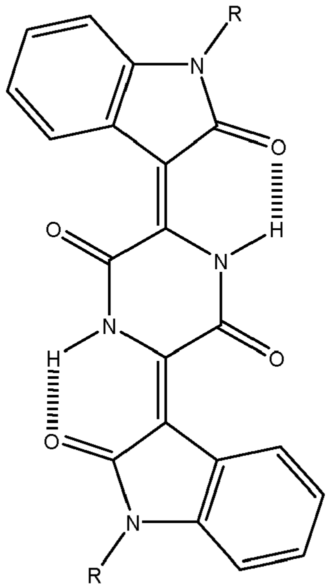

1

wherein R is be hydrogen (cpd. 21 a) or a methyl group (cpd. 21 b).

The compounds are obtained through the reaction of 1,4-diacetylpiperazine-2,5-dione and isatin.

Kunkel et al., J. Med. Chem. 2008, 51, 4563-4570 describe the antitumor activity of Bis-indole derivatives. Compounds 7 of this reference are obtained by reacting isatine and 1,4-diacetylpiperazine-2,5-dione and thus correspond to structure 21 of the aforementioned reference.

Caster et al., J. Heterocycl. Chem. 25, 591 (1988) describe conjugated systems derived from piperazine-2,5 dione. Compound 8 in this document resembles the structure 21 shown above. This document also discloses an alternative synthesis of these compounds by reacting 1,4-diacetylpiperazine-2,5-dione with 2-chloroindol-3-one in dimethylformamide at room temperature using triethylamine as a base.

Palenik et al., J. Heterocycl. Chem. 26, 821 (1989) relates to 2-chloroindol-3-one and its reaction with nucleophiles and discloses the same reaction as Caster et. al. referred to above.

US 2012/0232088 relates to piperazinedione compounds suitable for use as tyrosine kinase inhibitors for the treatment of cancer.

Some of the compounds comprise the structural element

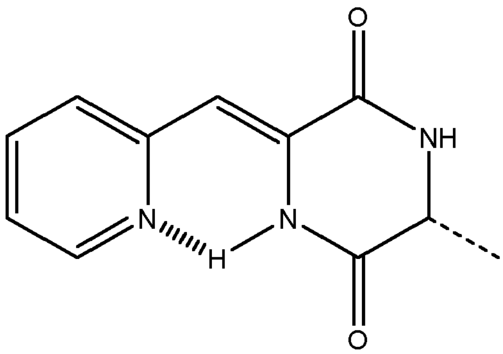

i.e. are capable of forming intramolecular hydrogen bonds.

None of the prior art documents cited above have a relation to organic electronics.

Meijer et. al, Chem. Eur. J. 2000, 6, No. 24, pp 4597-4603 relates to intramolecular hydrogen bonding in ladder like polymers and the effect of such hydrogen bonding on the push-pull character and the electron affinity of the oligomers. The oligomers are based on repeating units derived from symmetrical 2,5-bis(2-aminophenyl)pyrazines and comprise the common

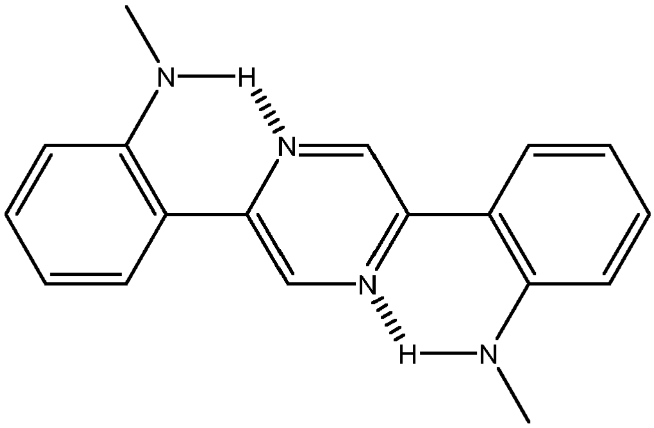

structural element

The authors come to the conclusion that the intramolecular hydrogen bonds support the planarization of the conjugated $\pi$-system in the molecules.

The oligomers are obtained by a Pd catalyst catalyzed coupling reaction.

Valiyaveettil and Vetrichetelvan, Chem. Eur. J. 2005, 11, 5889-5898 describe the intramolecular hydrogen bond-assisted planarization of asymmetrically functionalized alternating phenylene-pyridinylene copolymers. Structures P4 and P5 depicted below are said to be capable of forming intramolecular hydrogen bonds

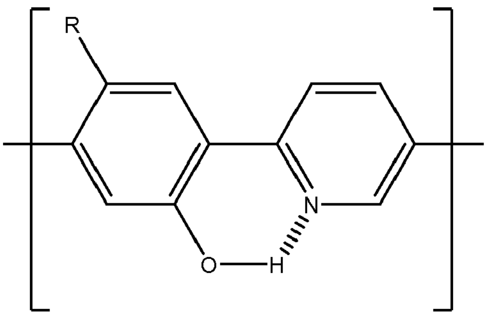

P4

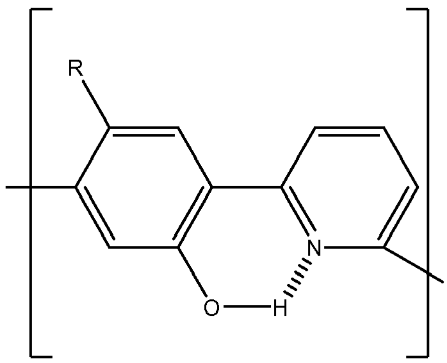

P5

The polymers are obtained by Suzuki polycondensation reactions using Pd compounds as catalysts.

The results provided show a red shift of the emission of the compounds showing intramolecular hydrogen bond formation in comparison with compounds where no such intramolecular hydrogen bond formation is possible.

Organic semiconductors are an important component in all kinds of various organic electronic devices like e.g. organic light emitting diodes OLED), organic field effect transistors (OFET) or organic photovoltaic (OPV) devices.

Accordingly, it was an object of the present invention to identify compounds which are particularly suitable as organic semiconductors for these applications and which can thus be used in such devices. The suitable compounds should be readily available and provide a good tuning ability of their electronic properties as well as their solubility properties.

This object has been achieved by the use in accordance with claim 1.

Furthermore, in accordance with other embodiments of the present invention, novel compounds suitable for use as organic semiconductors in organic electronic devices are provided.

Preferred embodiments of the present invention are set forth in the dependent claims and the detailed description hereinafter.

In a first embodiment, the present invention relates to the use of compounds comprising at least one of structural elements (1) to (5) or of oligomers or polymers comprising units obtained from compounds of formulas (1) to (5)

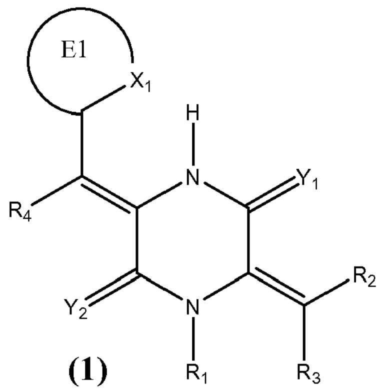

(1)

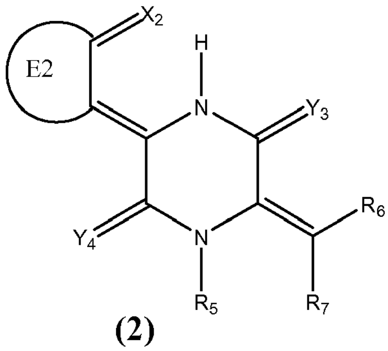

(2)

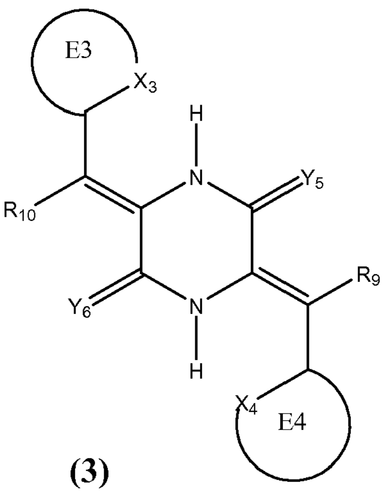

(3)

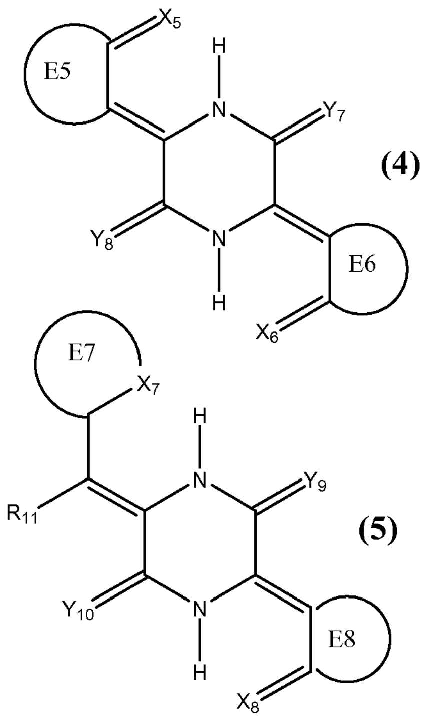

(4)

(5)

wherein

$X_1$, $X_3$, $X_4$ and $X_7$, which may be the same or different, represent hydrogen bond acceptors selected from the group consisting of -O-, -S-, -Se- and =N-, which hydrogen bond acceptors are part of rings E1, E3, E4 and E7, respectively,

$X_2$, $X_5$, $X_6$ and $X_8$, which may be the same or different, represent hydrogen bond acceptors selected from the group consisting of O, S, Se and N-$R_{12}$, which hydrogen bond acceptors are attached to rings E2, E5, E6 and E8, respectively, via a double bond,

$Y_1$ to $Y_{10}$, which may be the same or different, represent O, S, Se or $NR_{13}$,

E1 to E8, which may be the same or different, represent a 5- or 6-membered conjugated ring, which may be substituted or unsubstituted or fused with one or more conjugated rings, which may be substituted or unsubstituted, and

$R_1$ to $R_{13}$ represent hydrogen, a $C_1$-$C_{20}$- hydrocarbyl group or a $C_1$-$C_{20}$-heterohydrocarbyl group,

as organic semiconductors in organic electronic devices

Further embodiments of the present invention relate to certain novel compounds, oligomers and polymers and to organic electronic devices comprising compounds, oligomers or polymers comprising a structural element comprising a 2,5-disubstituted piperazine structural element.

Preferred organic electronic devices for the use in accordance with the present invention are organic light emitting diodes (OLED), organic field effect transistors(OFET), organic thin film transistors (OTFT) and organic photovoltaic devices (OPV).

It is apparent that the compounds, oligomers or polymers suitable for use in accordance with the present invention share a common structural element derived from a 2,5-disubstituted piperazine with hydrogen atoms bound to the nitrogen atoms of the piperazine ring being involved in an intramolecular hydrogen bonding. The important feature of the compounds are the disubstituted piperazine core and the involvement of this structural element in intramolecular hydrogen bonding as shown.

The term hydrogen bond, as used in the present invention, denotes an attractive interaction between polar molecules in which hydrogen is bound to a electronegative atom exceeding the electronegativity of carbon. Hydrogen bonds are stronger than van der Waals forces but weaker than covalent bonds. The hydrogen atom bound to the electronegative atom is generally referred to as a hydrogen bond donor whereas an electronegative atom having a free electron pair to share with the hydrogen atom is the so called hydrogen bond acceptor.

Due to the hydrogen atom being bound to an electronegative atom, and in view of the small size of the hydrogen atom, a comparatively large positive partial charge density is observed. The hydrogen bond occurs if this strong positive charge density attracts a lone pair of electrons on another electronegative heteroatom.

$X_1$ to $X_8$ in the formulas (1) to (5) represent the hydrogen bond acceptor and are selected from O,S, Se or N, preferably from O, S or N and particularly preferably from S or N.

$X_1$, $X_3$, $X_4$ and $X_7$ form part of the ring systems E1, E3, E4 and E7, respectively. This means that the rings E1, E3, E4 and E7 contain a subunit -O-, -S-, -Se- or =N-. Preferably, $X_1$, $X_3$, $X_4$ and $X_7$, which may be the same or different, are -S- or =N-.

$X_2$, $X_5$, $X_6$ and $X_8$ are attached to the respective ring systems E2, E5, E6 and E8 and thus these ring systems comprise a substituent =O, =S, =Se or =$NR_{12}$, where $R_{12}$ is a hydrogen atom, a $C_1$-$C_{20}$-hydrocarbyl group or a $C_1$-$C_{20}$-heterohydrocarbyl group. Preferably $R_{12}$ is a hydrogen atom or a $C_1$-$C_{20}$-hydrocarbyl group, in particular a $C_6$-$C_{20}$ linear or branched alkyl group.

Hydrogen bond acceptors $X_1$ to $X_8$ form hydrogen bonds with the hydrogen atoms attached to the nitrogen atoms of the piperazine ring in the structure. In formulas (1) and (2) one hydrogen bond is formed whereas in structures (3) to (5) two hydrogen bonds are established.

$Y_1$ to $Y_{10}$, which may be the same or different are bound to the piperazine ring via a double bond and represent preferably O or S, in particular O. In accordance with a preferred embodiment $Y_1$=$Y_2$, $Y_3$=$Y_4$, $Y_5$=$Y_6$, $Y_7$=$Y_8$ and $Y_9$=$Y_{10}$, i.e. the two substituents Y in the same piperazine ring are preferably identical.

E1 to E8, which might be the same or different, represent a 5- or 6-membered conjugated ring, which may be substituted or unsubstituted or fused with or bound to one or more additional conjugated rings, which may be substituted or unsubstituted.

The term "conjugated ring" when used herein, denotes any 5-or 6-membered ring comprising a first one double bond in conjugation with a second other double bond, wherein the first and the second double bond may be within the same ring or wherein the first double bond may be within a ring and the second double bond might be outside the said ring. Aromatic or heteroaromatic rings having an aromaticity as defined in the Hückel ($4\pi$+2) rule are preferred examples of rings E1 to E8. The skilled person knows the respective rings and will select the most appropriate rings for E1 to E8 based on his professional experience. Accordingly, no further details need to be given here.

As can be seen from formulas (1) to (5), the rings E1, E3, E4 and E7 comprise as ring atom the hydrogen bond acceptor groups $X_1$, $X_3$, $X_4$ and $X_7$ and thus these rings comprise at least one heteroatom. Rings E1, E3, E4 and E7 are attached to the piperazine ring through a spacer of one carbon atom as can be seen from formulas (1), (3) and (5) whereas rings E2, E5, E6 and E8 are directly attached to the piperazine ring through a double bond. The hydrogen bond acceptors $X_2$, $X_5$, $X_6$ and $X_8$, respectively, are bound to rings E2, E5, E6 and E8, respectively, through a double bond but does not form part of rings E2, E5, E6 or E8.

A preferred group of compounds, polymers or oligomers for use in accordance with the invention are those

comprising structural elements (1) to (5) wherein $X_3=X_4$ or $X_5=X_6$.

The substituents $R_1$ to $R_{13}$ represent a hydrogen atom, a $C_1$-$C_{20}$-hydrocarbyl group or a $C_1$-$C_{20}$-heterohydrocarbyl group.

Hydrocarbyl groups are obtained by removing one hydrogen atom from a hydrocarbon, which may be saturated or unsaturated, linear or branched. Preferred hydrocarbyl groups in the formulas (1) to (5) are alkyl groups, which may be linear or branched. Preferably these alkyl groups comprise of from 6 to 20 carbon atoms

A heterohydrocarbyl group is obtained by replacing one carbon atom of a hydrocarbyl group by a heteroatom, preferably a heteroatom selected from the group consisting of O, S, N, P or Si. Preferred heterohydrocarbyl groups are heteroalkyl groups having 4 to 20 carbon atoms.

A first preferred group of compounds, oligomers and polymers for use in accordance with the present invention comprise structural elements of formula (6) or units obtained from formula (6)

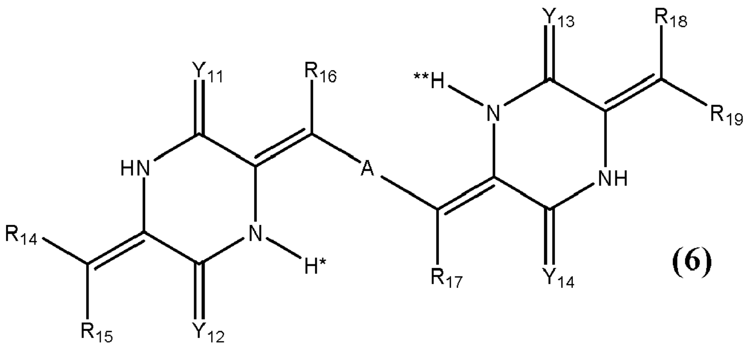

(6)

wherein $Y_{11}$ to $Y_{14}$, which may be the same or different, represent O, S Se or $NR_{20}$, preferably O or S, , $R_{14}$ to $R_{20}$ represent hydrogen, a $C_1$-$C_{20}$ hydrocarbyl group or a $C_1$-$C_{20}$ heterohydrocarbyl group, preferably a hydrogen atom or a linear or branched alkyl group with 6 to 20 carbon atoms, and

A represents a bidentate linking group selected from 6-membered heteroaryl rings or from systems comprising at least two connected or fused 5- or 6-membered conjugated rings, which may be substituted or unsubstituted, comprising two hydrogen bond acceptor groups selected from O, S. Se or N, preferably O, S or N, in the 6-membered heteroaryl ring or comprising at least one hydrogen bond acceptor group selected from O, S. Se or N, preferably O, S or N, in at least two of the connected or fused rings which form hydrogen bonds with the two hydrogen atoms H* and H**.

Preferred groups A are selected from the group consisting of formulas (7) to (25)

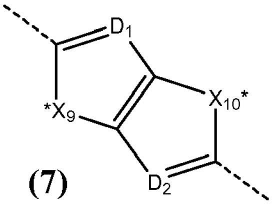

(7)

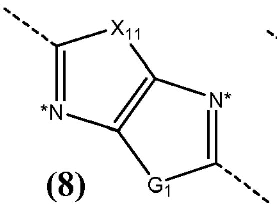

(8)

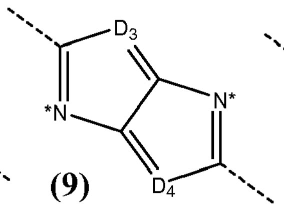

(9)

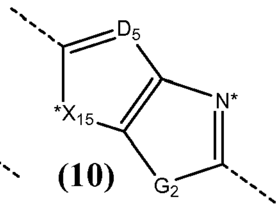

(10)

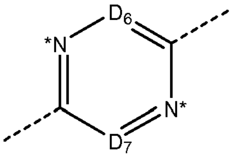

(11)

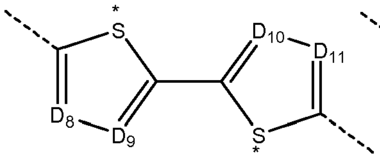

(12)

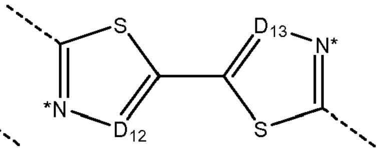

(13)

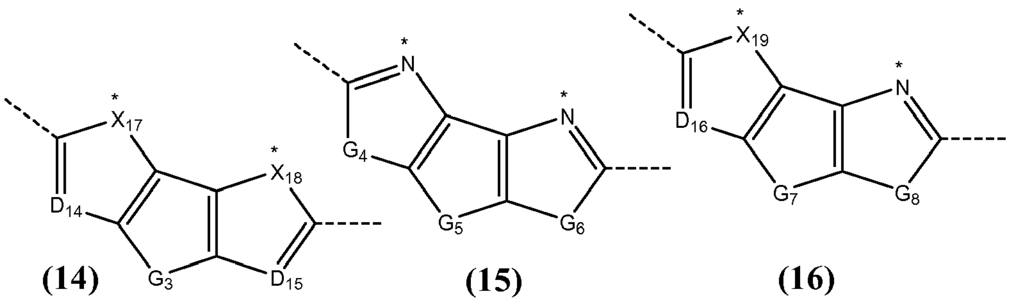

(14) (15) (16)

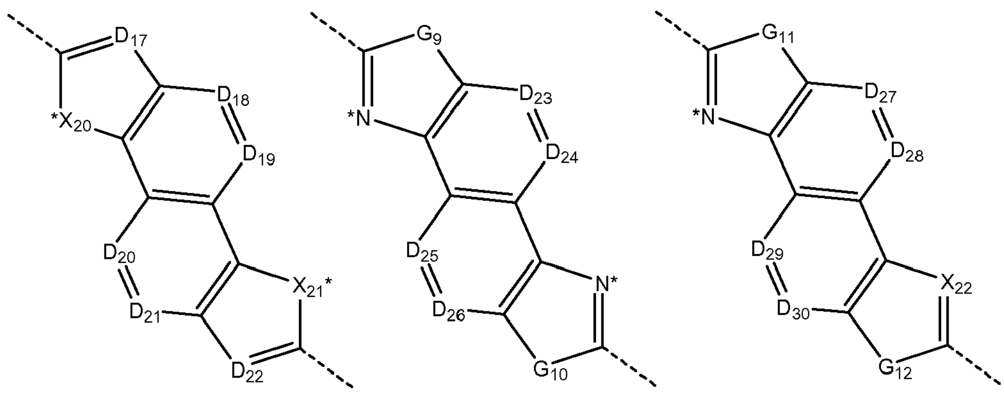

(17) (18) (19)

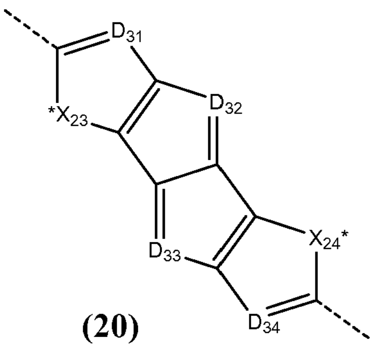

(20)

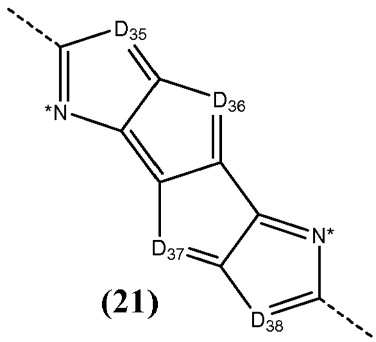

(21)

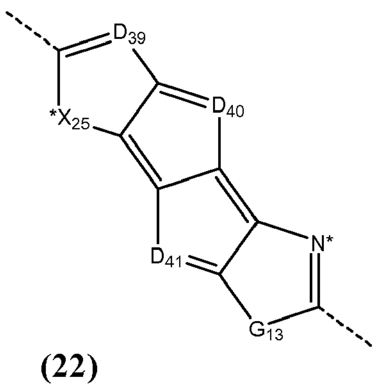

(22)

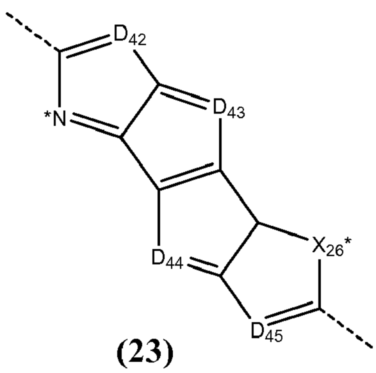

(23)

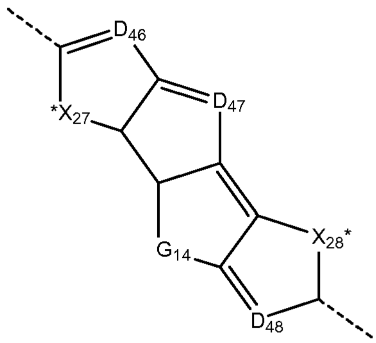

**(24)**

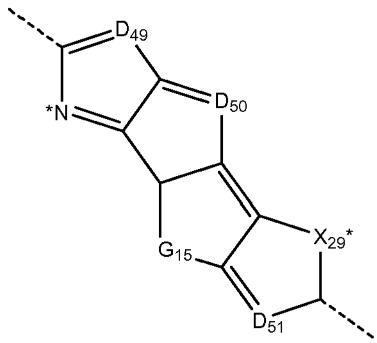

**(25)**

wherein
* denotes the hydrogen bond acceptor groups forming the hydrogen bond with H* and H** in formula (6),
the dashed line represents the linking atoms to the remainder of the molecule,
$D_1$ to $D_{51}$, which may be the same or different, represent N or $CR_{21}$, $G_{12}$ to $G_{15}$, which may be the same or different, represent O, S, Se, $NR_{22}$, C(=O) C(=S), $C(CR_{23}R_{24})$,
$R_{21}$ to $R_{25}$, which may be the same or different, represent hydrogen, a $C_1$-$C_{20}$ hydrocarbyl group or a $C_1$-$C_{20}$ heterohydrocarbyl group, preferably a hyrogen atom or a linear or branched alkyl group with 6 to 20 carbon atoms, and
$X_9$ to $X_{29}$, which may be the same or different, represent a hydrogen bond acceptor group selected from -O-, -S-, -Se- or $-NR_{25}$, preferably O or S.

Preferred 6-membered heteroaryl groups are

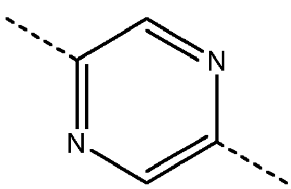

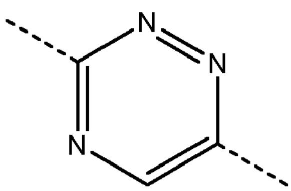

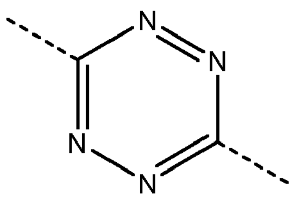

wherein the piperazine ring is particularly preferred. The 6-membered ring may be substituted or unsubstituted.

Some preferred examples for compounds of formula (6) wherein A is represented by one of formulas (7) to (25) are shown below

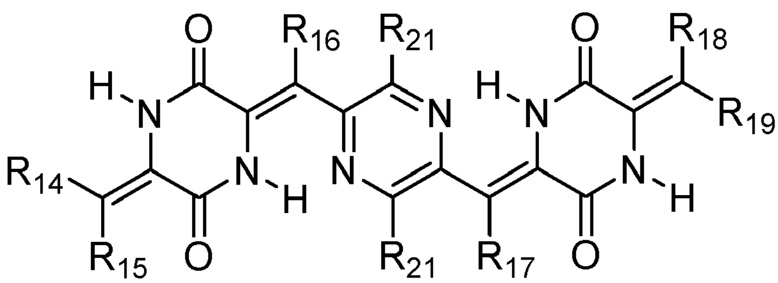

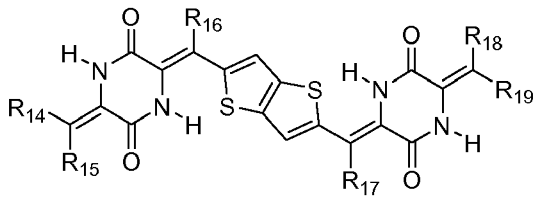

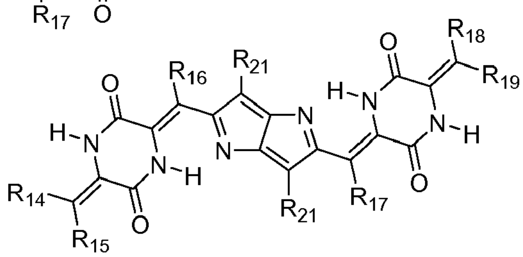

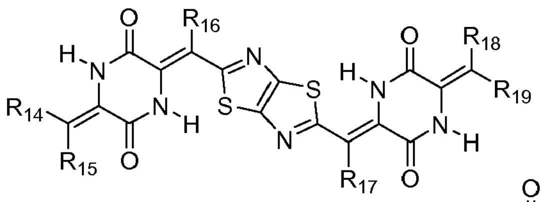

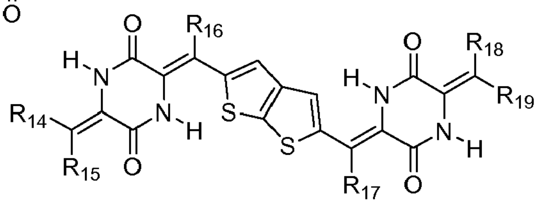

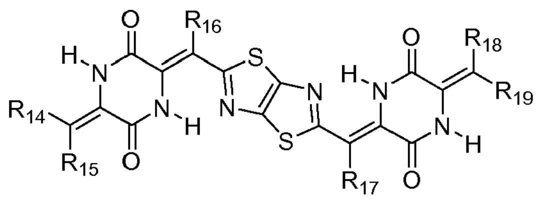

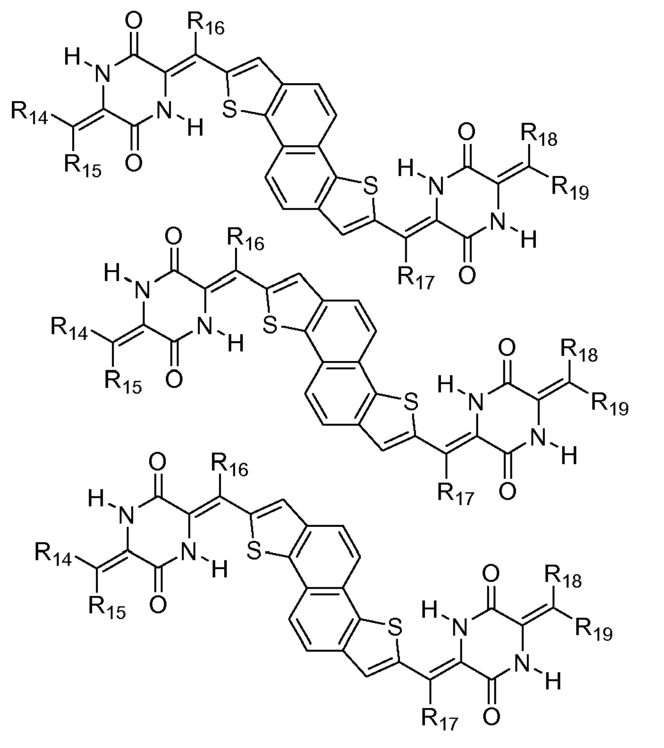

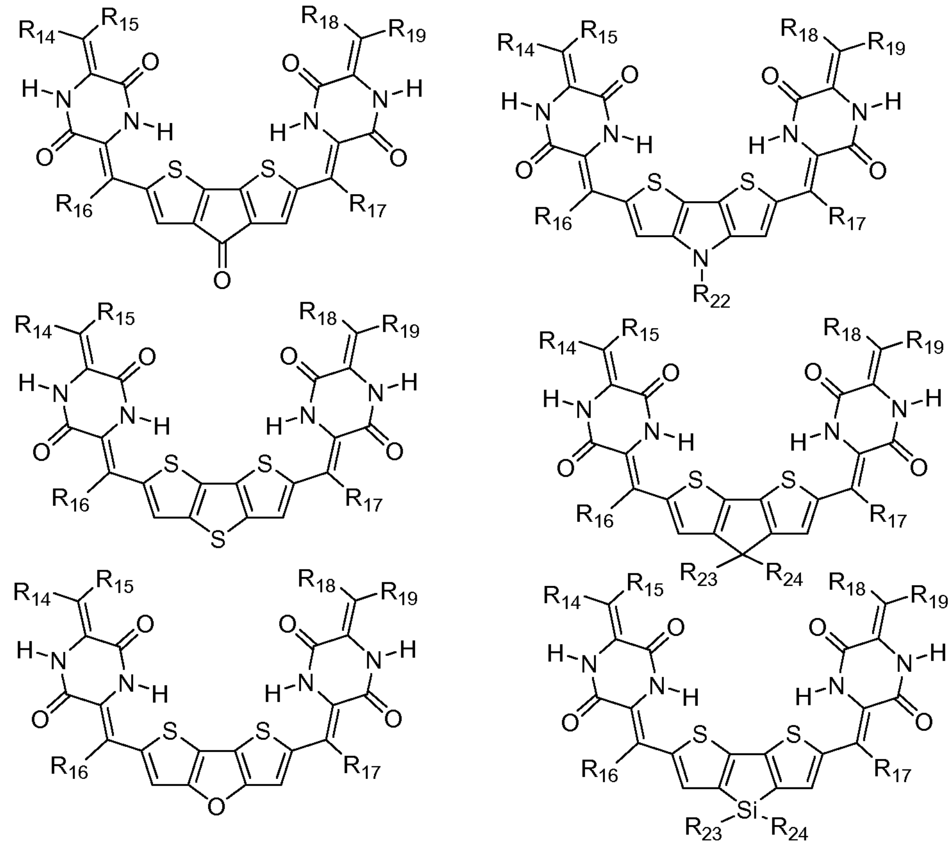

where $R_{14}$ to $R_{19}$, and $R_{21}$, $R_{23}$ and $R_{24}$ may have the meanings defined above and preferably are a linear or branched alkyl group having 6 to 20 carbon atoms or a hydrogen atom.

In another preferred group of compounds of formula (6) for use in the present invention A represents a bidentate linking group of formulas (26) to (28)

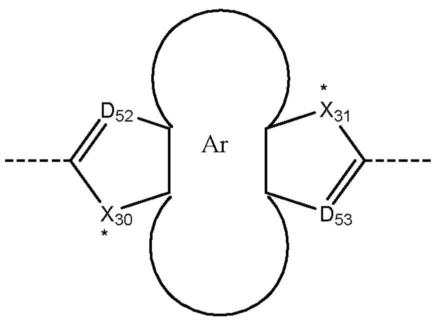

**(26)**

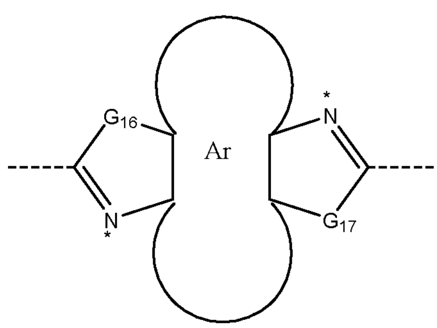

**(27)**

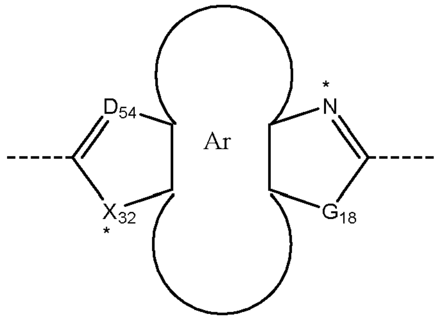

**(28)**

wherein
* denotes the hydrogen bond acceptor groups forming the hydrogen bond with H* and H** in formula (6),
the dashed line represents the linking atoms to the remainder of the molecule,
$D_{52}$ to $D_{54}$, which may be the same or different, represent N or $CR_{26}$, $G_{16}$ to $G_{18}$, which may be the same or different, represent O, S, Se, $NR_{27}$, C(=O) C(=S)or $C(CR_{28}R_{29})$,
$R_{26}$ to $R_{30}$, which may be the same or different, represent hydrogen, a $C_1$-$C_{20}$ hydrocarbyl group or a $C_1$-$C_{20}$ heterohydrocarbyl group, preferably a linear or branched alkyl group with 6 to 20 carbon atoms or a hydrogen atom, and
$X_{30}$ to $X_{32}$, which may be the same or different, represent a hydrogen bond acceptor group selected from -O-, -S-, -Se- or $-NR_{30}$, preferably O or S. wherein Ar represents a six membered aromatic or heteroaromatic ring or an acene skeleton having two to 6 fused aromatic or heteroaromatic 6-membered rings which may be substituted or unsubstituted.

Just by way of example some compounds comprising a linking group A in accordance with formulas (26) to (28) are given below

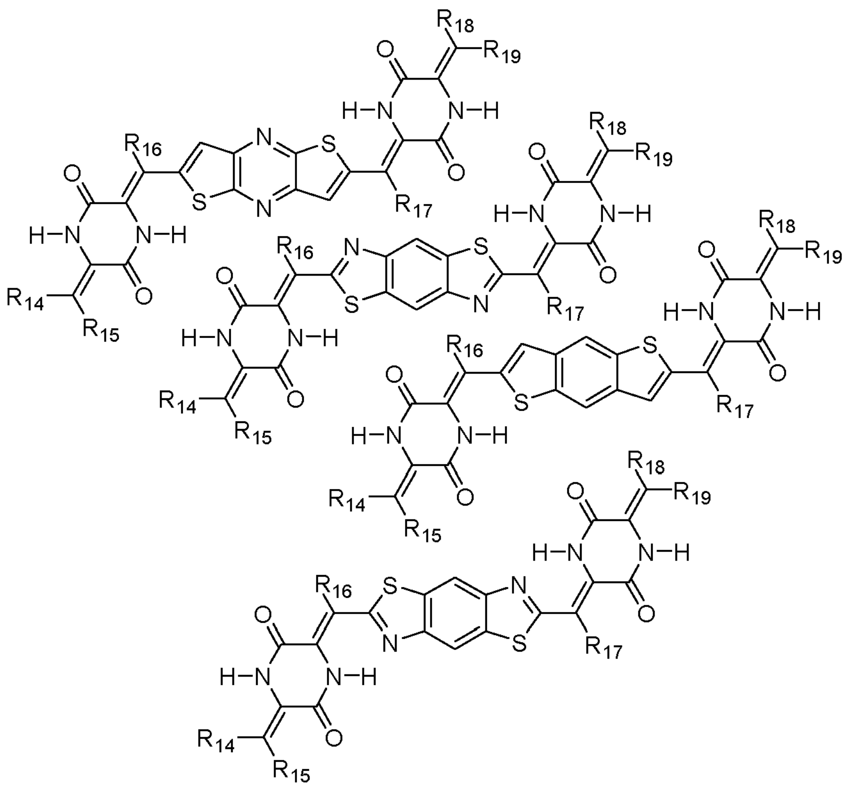

wherein the substituents are as defined above.

The following examples show preferred compounds of formulas (29) to (34) which have been found particularly useful as organic semiconductors in organic electronic devices under certain circumstances.

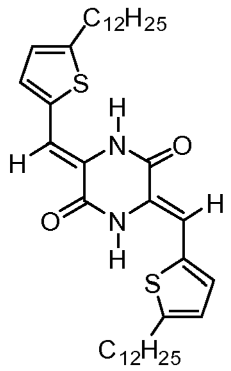
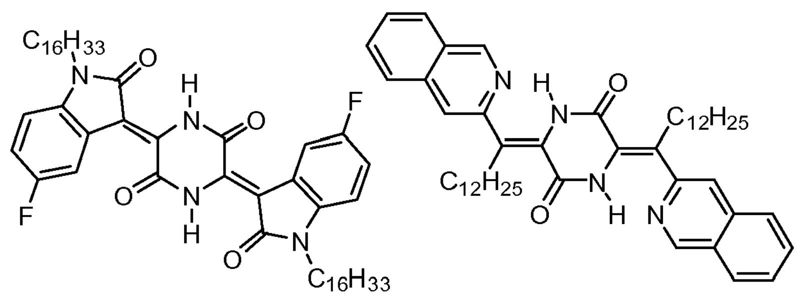

(29) (30) (31)

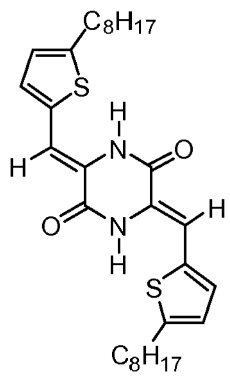
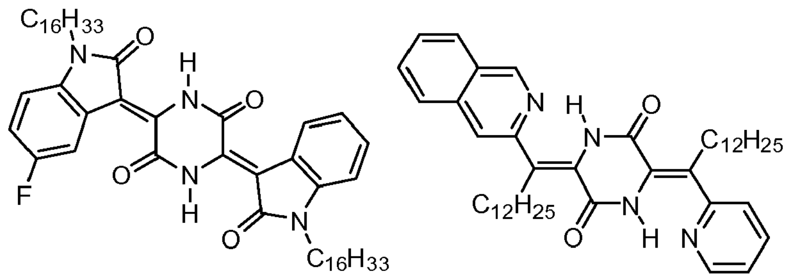

(32) (33) (34)

Further preferred compounds for use in accordance with the present invention are the following compounds of formulas (35) to (40)

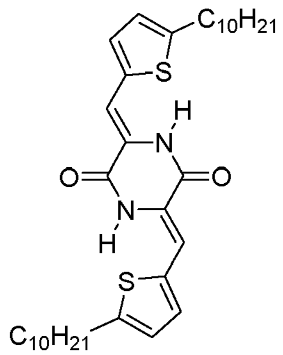
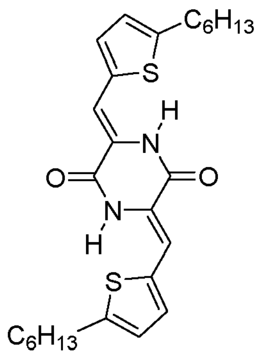
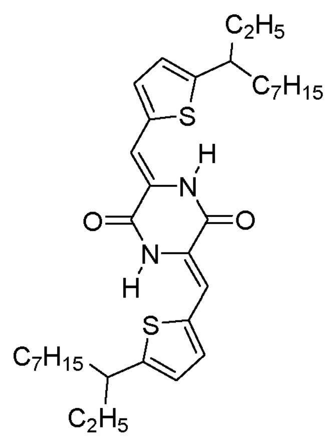

(35) (36) (37)

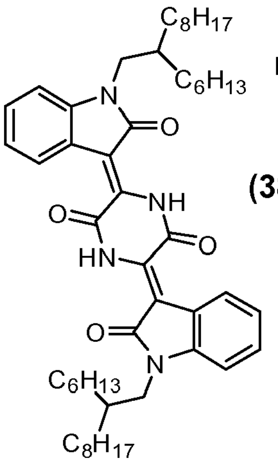
(38)

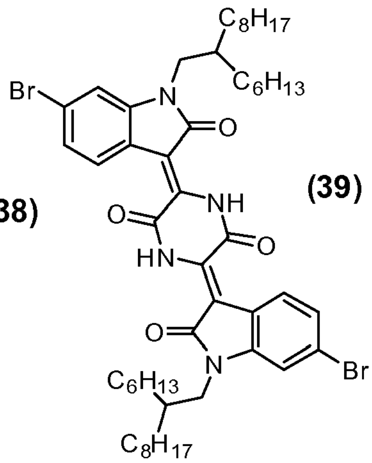
(39)

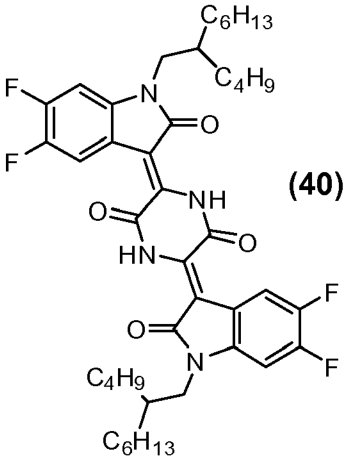
(40)

Some further examples for preferred compounds for use in accordance with the present invention are compounds (41) to (44) presented below

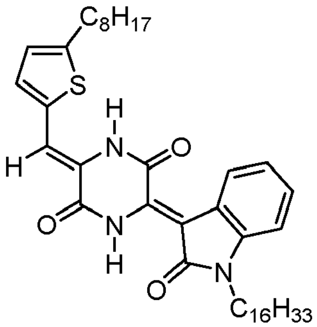
(41)

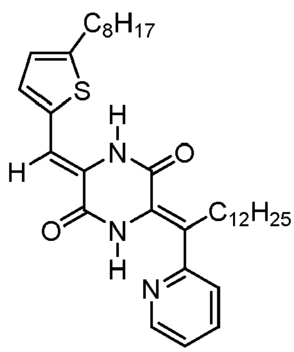
(42)

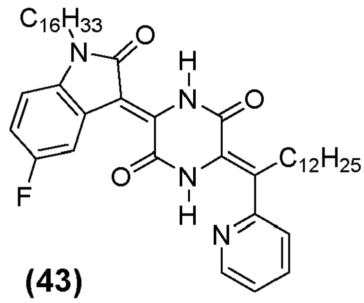
(43)

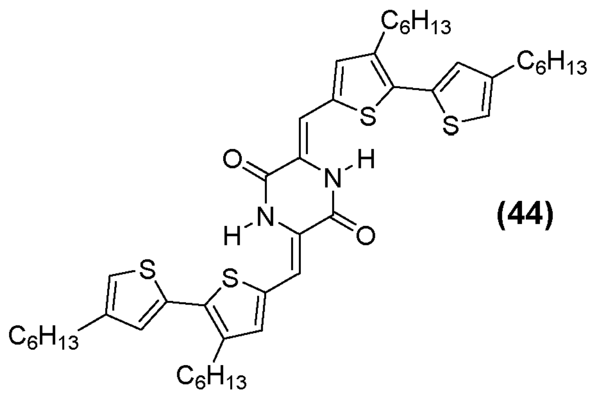
(44)

The compounds of formula (6) or oligomers or polymers obtained from compounds of formula (6), including compounds, oligomers or polymers wherein A is represented by any of formulas (7) to (28) are as such novel and represent another embodiment of the present invention.

Preferred compounds, oligomers and polymers of this group are as defined and discussed above in connection with the use in accordance with the present invention.

A further preferred embodiment of the present invention relates to the use of compounds of formula (6) or oligomers or polymers comprising repeating units of formula (6) represented by formulas (45) and (46) as organic semiconductors in organic electronic devices and to such compounds, oligomers and polymers as such

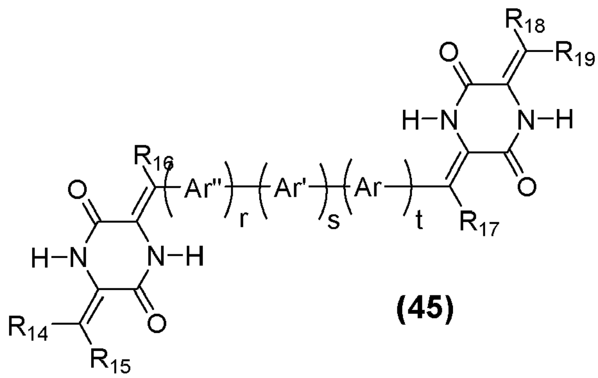

**(45)**

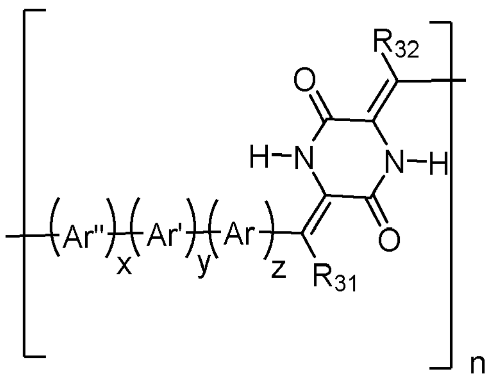

**(46)**

wherein

Ar and Ar'' represent 5- or six membered heteroaryl groups, which may be substituted or unsubstituted and which may form part of a fused ring system and which comprise at least one hydrogen bond acceptor group selected from O, S. Se or N which is capable of forming a hydrogen bond with the hydrogen atoms of the diketopiperazine group,
Ar' is an aryl or heteroaryl group with 1 to 30 carbon atoms, which may be substituted or unsubstituted and which may form part of a fused ring system,
$R_{14}$ to $R_{19}$ are as defined above,
$R_{31}$ and $R_{32}$, which may be the same or different, represent hydrogen, a $C_1$-$C_{20}$-hydrocarbyl group or a $C_1$-$C_{20}$-heterohydrocarbyl group,
r, s and t, independent of one another, are an integer of from 0 to 5, the sum of r and t being at least 2,
x, y and z, independent of one another, are an integer of from 0 to 5, the sum of x and z being at least 2, and
n is an integer greater 1.

Some examples for compounds of formula (45) are shown below

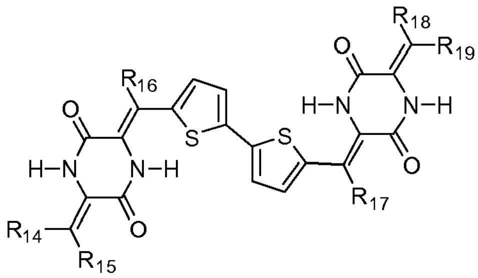

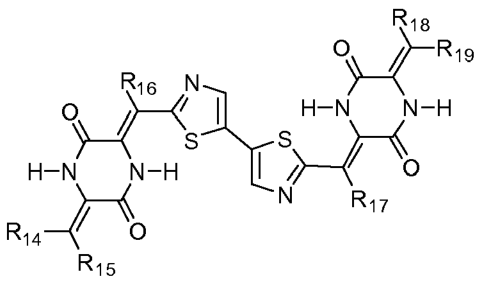

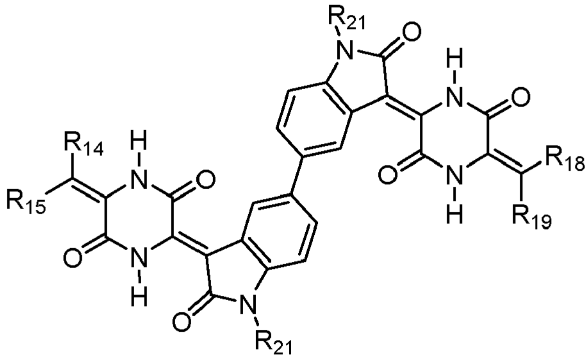

wherein $R_{14}$ to $R_{19}$ are as defined above and preferably $R_{14}$, $R_{15}$, $R_{18}$ and $R_{19}$ are linear or branched alkyl groups having form 6 to 20 carbon atoms.

The compounds of formula (45) or the oligomers and polymers comprising repeating units represented by formula (46) can be in principle obtained by a Knoevenagel type condensation of a 2,5-disubstituted piperazine compound with a di(arylaldehyde), di(heteroarylaldehyde), di(alkylarylketone) or di(alkylheteroarylketone.

The suitable reaction conditions for such Knoevenagel type condensations are known to the skilled person and need not be further detailed here.

In accordance with another preferred embodiment of the present invention, the compounds in accordance with formula (45) are modified to the effect that $R_{14}$ and $R_{18}$ are replaced by heteroaryl groups $Ar_4$ (which may be the same or different) which may be substituted or unsubstituted and which can be fused with one or more additional aryl or heteroaryl groups and which contain hydrogen bond acceptors selected from O, S, Se and N in a position to interact with the remaining NH groups of the piperazine group.

These compounds may be represented by the formula

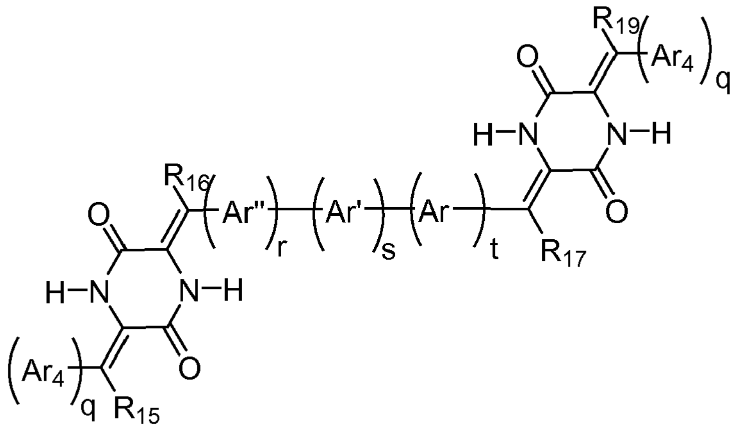

wherein q is an integer of from 0 to 5, preferably of from 1 to 5 and $R_{15}$, $R_{16}$, $R_{17}$, $R_{19}$, r, s and t have the meaning defined above.

Another preferred group of compounds for use in accordance with the present invention is represented by formula (47)

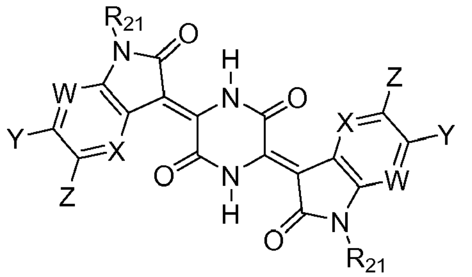

**(47)**

wherein

W and X which may be the same or different are CH or N, Y and Z which may be the same or different are hydrogen, halogen, cyanide or a $C_1$-$C_{20}$ hydrocarbyl or $C_1$-$C_{20}$ heterohydrocarbyl group or Y and Z form together a substituted or unsubstituted five or six membered carbocyclic or heterocyclic, aromatic or heteroaromatic ring, which ring may be part of a fused ring system, and

$R_{21}$ is as defined above.

Exemplary compounds of formula (47) are the following

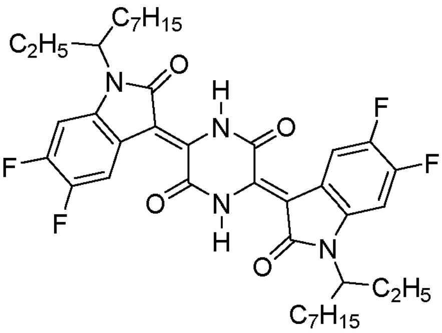

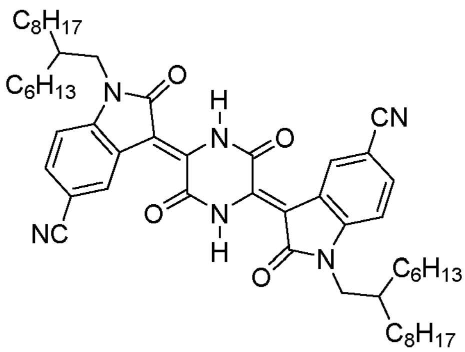

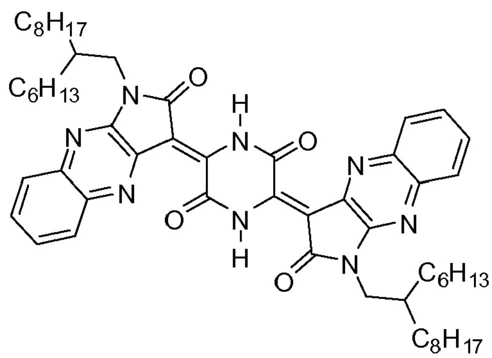

Oligomers or polymers of formula (48) or (49) are novel and are also suitable for use in accordance with the present invention.

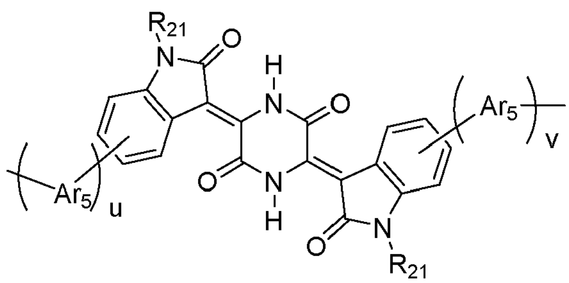

**(48)**

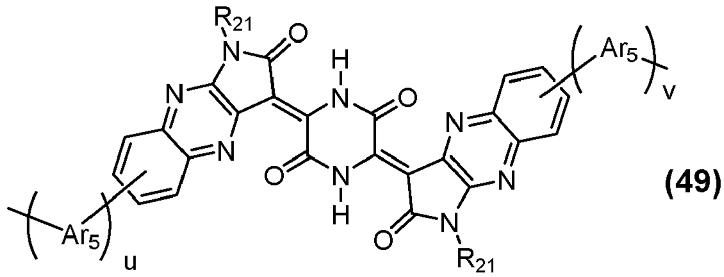

**(49)**

wherein $Ar_5$, which may be the same or different at each occurrence, represents a substituted or unsubstituted five of six membered aromatic or heteroaromatic ring, which ring may be part of a fused ring system, and u and v, independent of another may be 0 or an integer of 1 to 5 and $R_{21}$ is as defined above.

Oligomers and polymers for use in accordance with the present invention which can be obtained by a Knoevenagel type condensation are given as examples below

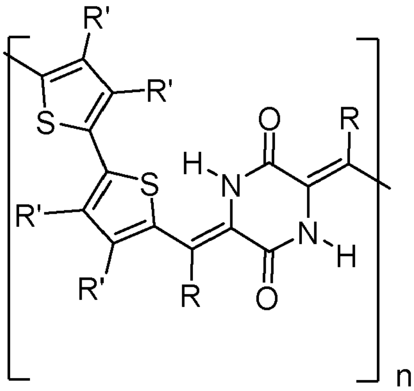

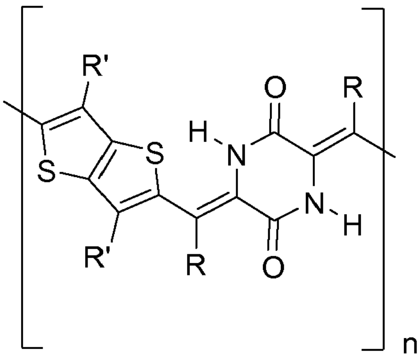

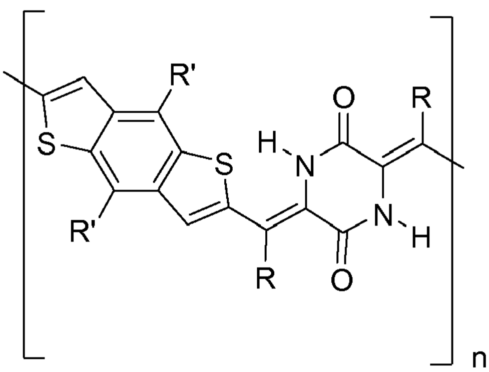

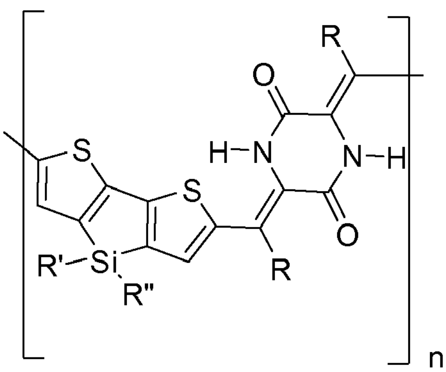

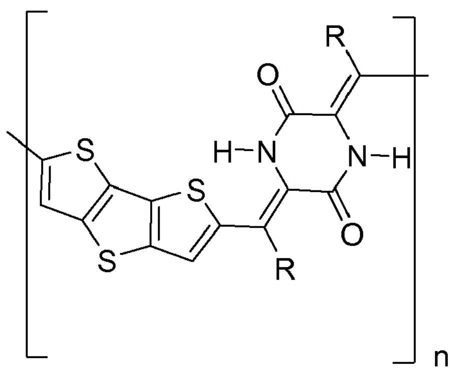

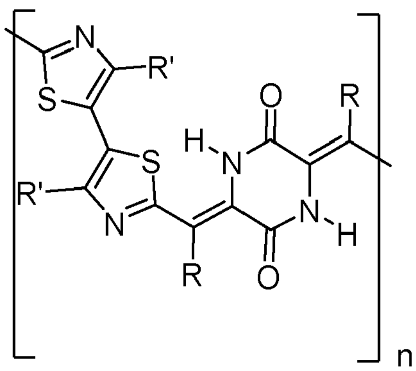

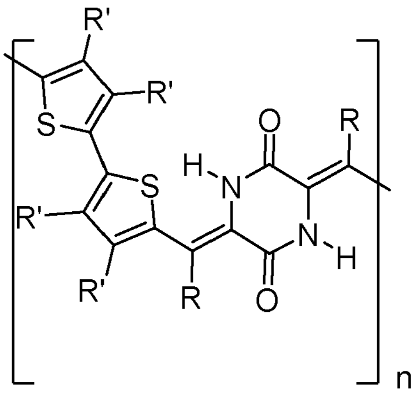

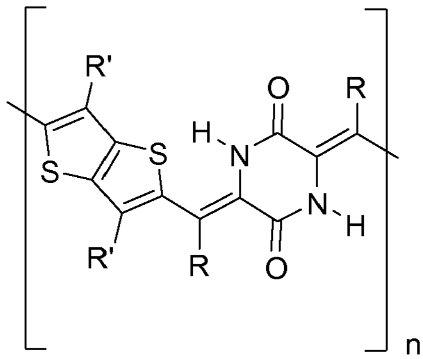

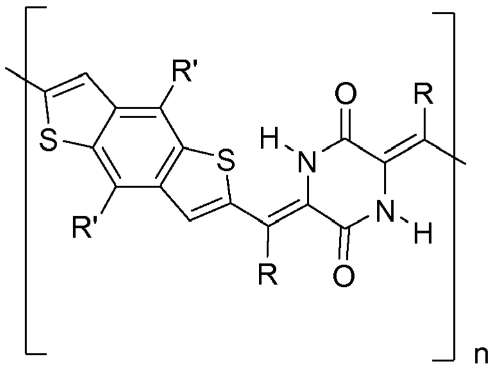

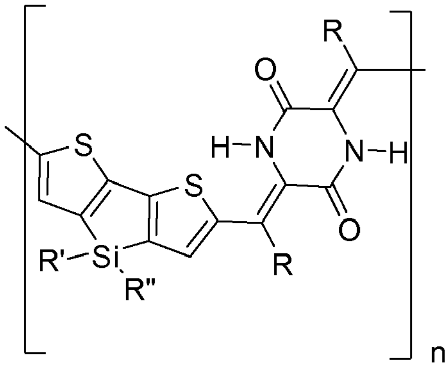

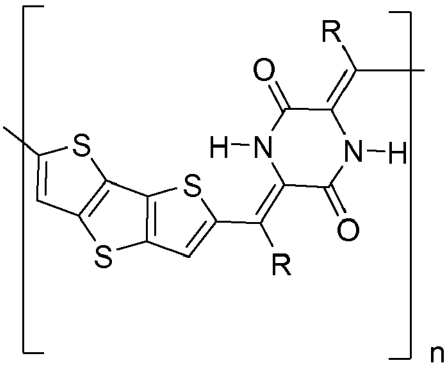

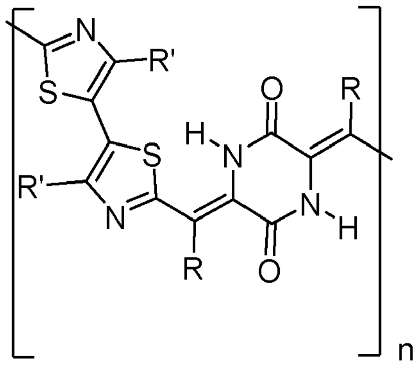

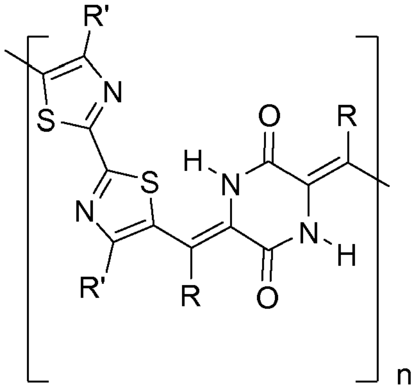

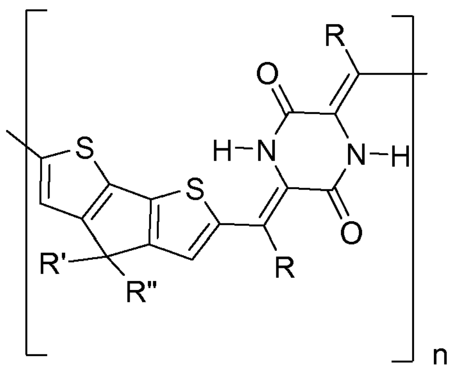

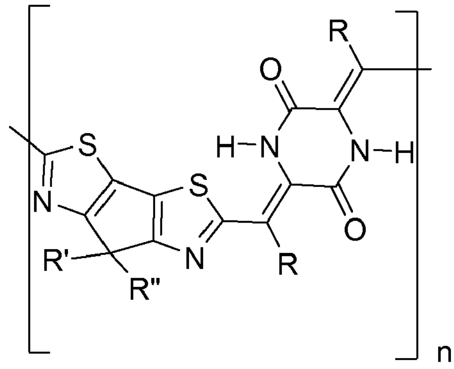

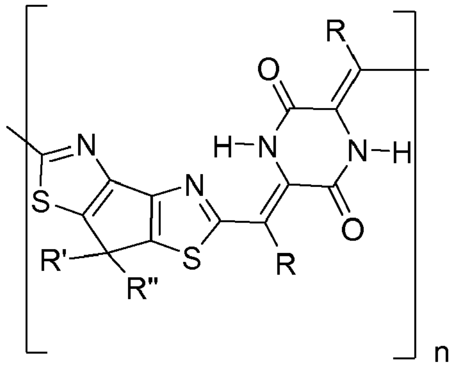

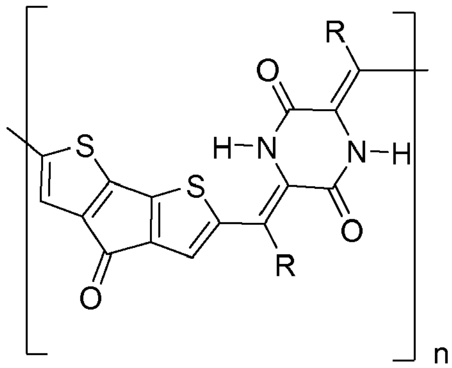

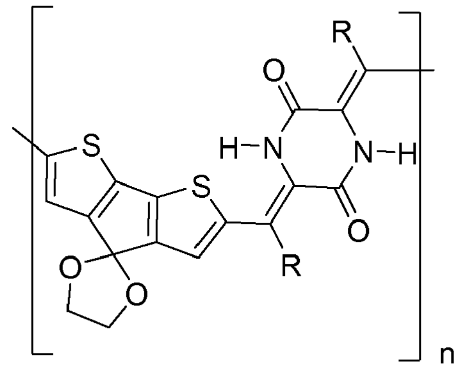

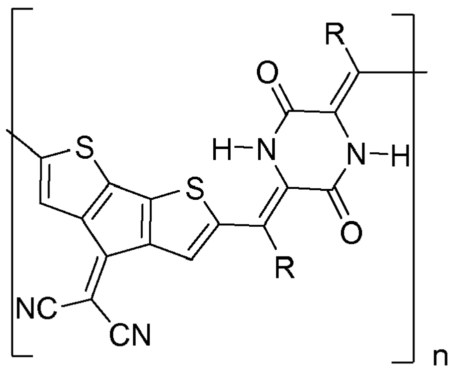

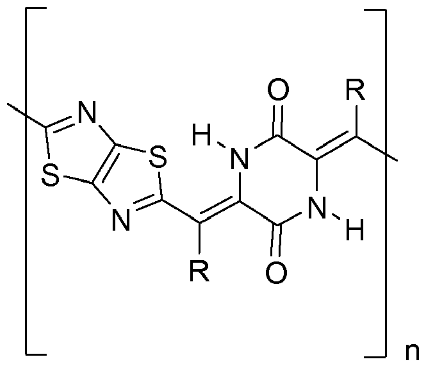

wherein n, which may be the same or different at each occurrence, represents an integer greater than 1 and R and R', which may be the same or different at each occurrence, may be hydrogen, a $C_1$-$C_{20}$ hydrocarbyl or a $C_1$-$C_{20}$ heterohydrocarbyl group, preferably a hydrogen atom or a linear or branched alkyl group with 6 to 20 carbon atoms.

The synthesis of the compounds, oligomers and polymers for use in accordance with the present invention can be obtained by synthesis methods known per se which have been described in the literature and which are known to the skilled person.

By way of example some principal reaction schemes for the synthesis of the compounds, oligomers or polymers which can be used in accordance with the present invention are given hereinafter.

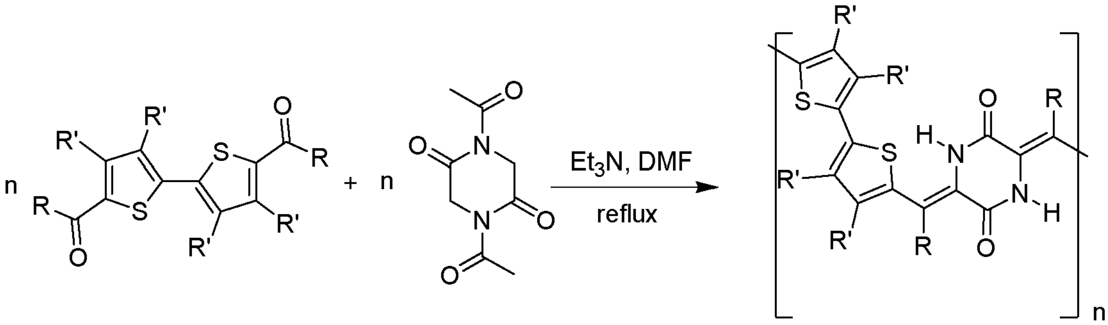
n
n
Et3N, DMF
reflux

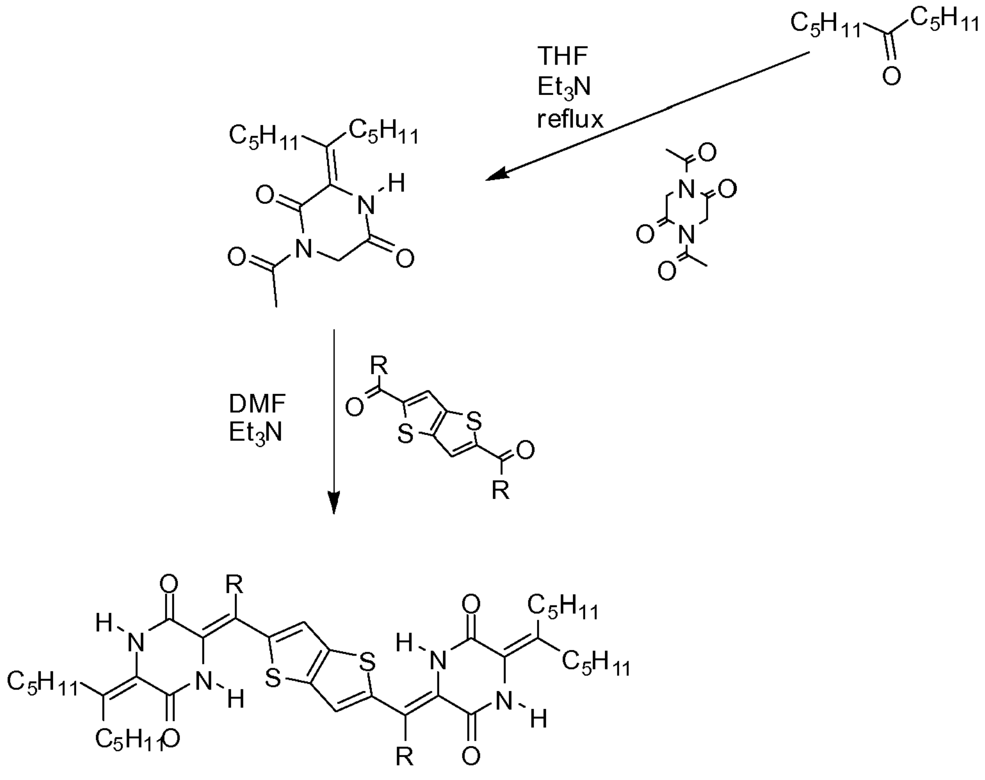
THF
Et3N
reflux
DMF
Et3N

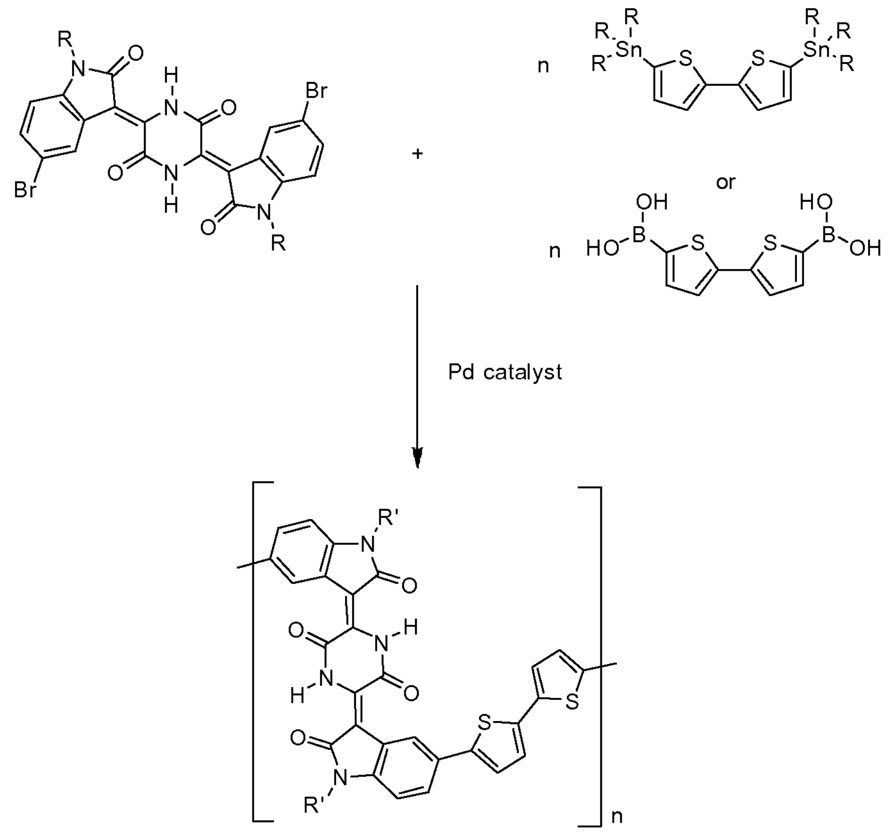
Pd catalyst

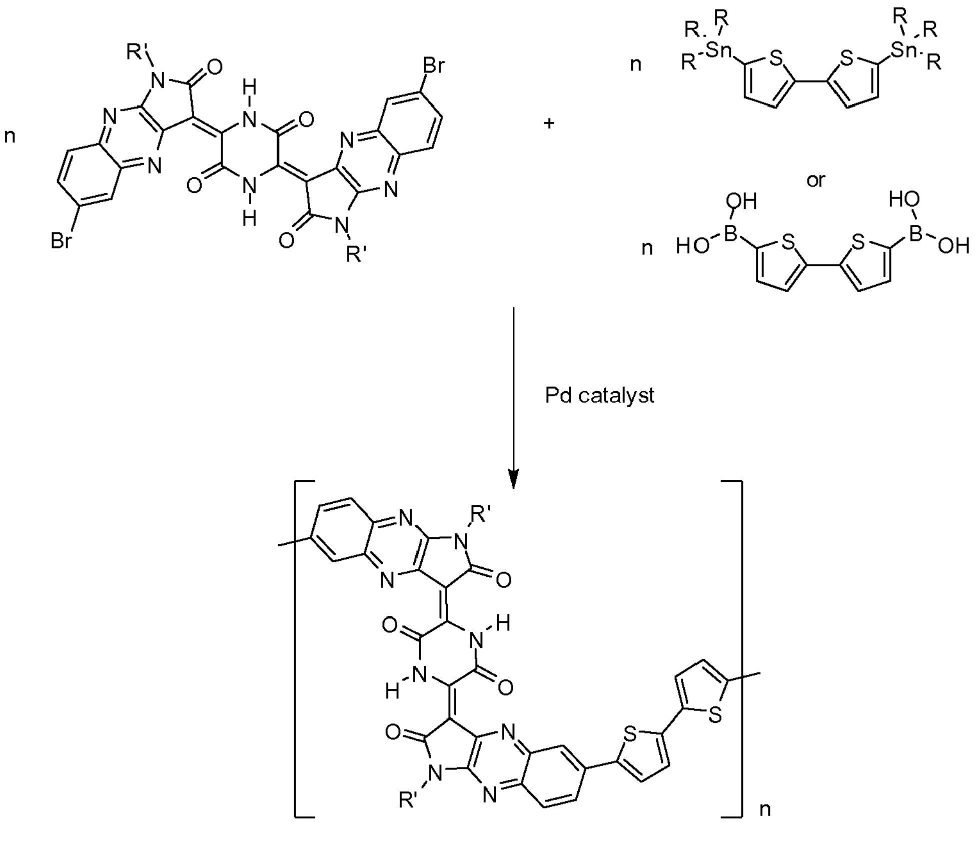
Pd catalyst

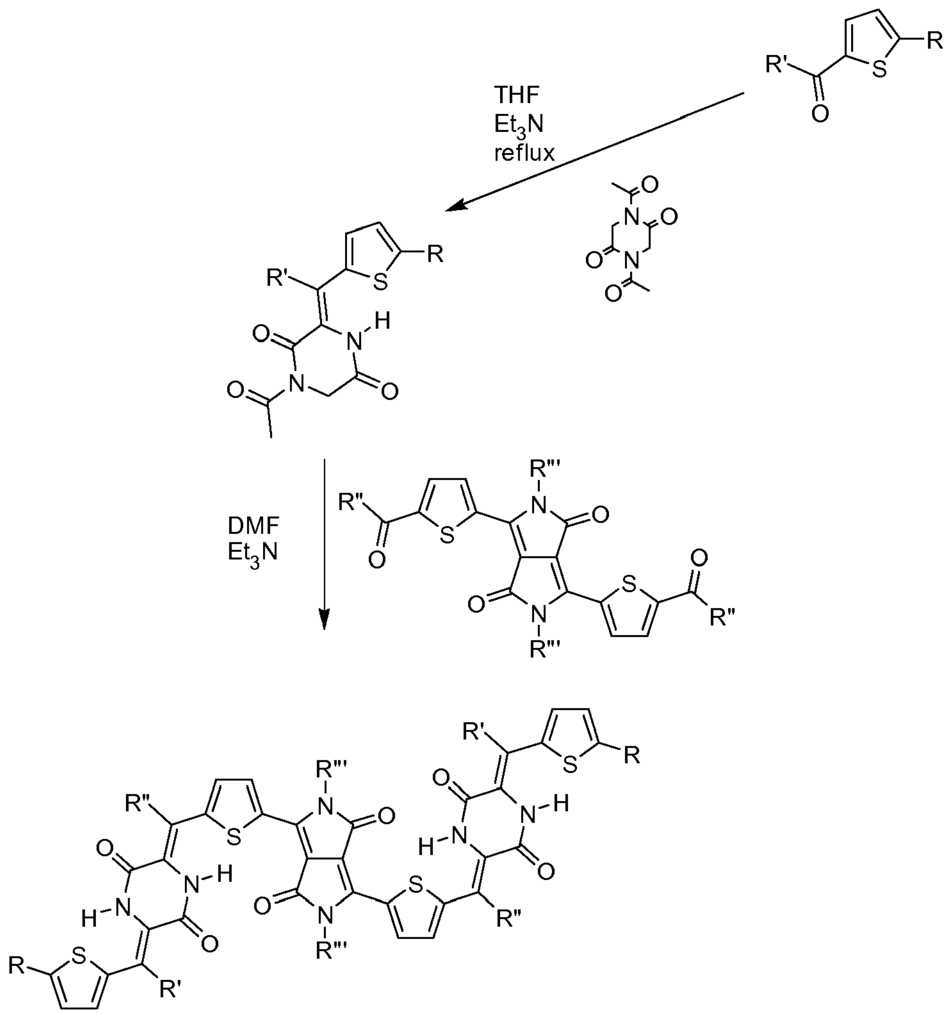


In accordance with the present invention, the compounds, oligomers and polymers described in detail hereinbefore are used as semiconductors in organic electronic devices, in particular organic light emitting diodes (OLED), organic field effect transistors (OFET), organic thin film transistors (OTFT) or organic photovoltaic devices (OPV).

The compounds, oligomers and polymers defined above may form one or more layers of such organic electronic devices or may constitute a component of such layers in such devices.

The compounds in accordance with the present invention are particularly suitable as efficient organic semiconductors, either in the form of small molecules or as building blocks in polymeric architectures.

By appropriate substitution of the conjugated cores with electron withdrawing groups the HOMO and LUMO of the compounds can be tuned in accordance with the specific needs of the individual application. By grafting various chains the solubility and the $\pi$-stacking can be promoted and the organic semiconducting properties generally improved.

The mobility of the compounds in accordance with the present invention is improved compared to compounds known from the prior art.

Due to the good solubility of the compounds in accordance with the present invention, thin films can be prepared by e.g. spin coating out of different solvents with good reproducibility.

High performance organic semiconductors can advantageously be used for flexible electronic applications like e.g. display backplanes or RFID tags. Other examples are so called E-readers.

The mobility required for such devices has to reach certain minimum values and the compounds or the compositions in accordance with the present invention show good mobilities and thus can form the basis for the development of respective organic electronic devices.

**Examples**

**Example 1 -** Synthesis of 2-decylthiophene

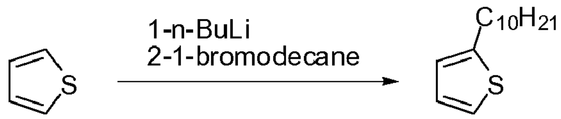


In a dried 1 liter round bottom flask maintained at a temperature between 0 and -10°C, were introduced under Argon 240ml of anhydrous THF and 208ml (0.334mol) of n-Buli 1.6M in hexane. Then 26.7ml (0.331 mol) of thiophene were gently added while keeping the temperature between 0 and -10°C. Ice salt bath was removed and the mixture was stirred until reaching room temperature. 78ml (0.368mol) of 1-bromodecane were added and the mixture was stirred for 2 h at room temperature then overnight under reflux. Afterwards, the reaction medium was poured in 1 L of ice water under vigorous stirring. Organics were extracted with t-butylmethylether and dried over magnesium sulphate. After removing the solvent under reduced pressure, 78.8g of crude product were purified by distillation under vacuum (0,1mbar, 95-105°C) to give 45.3g (0.202mol) of pure product (yield 61 %).

**Example 2** - Synthesis of 5-hexylthiophene-2-carbaldehyde

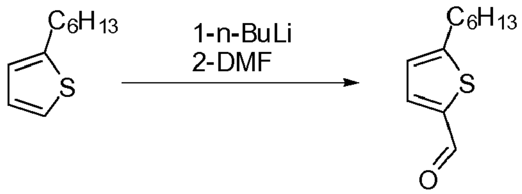


In a 250ml three necked round bottom flask were introduced under argon 6.08g (35mmol) of 2-hexylthiophene in 80ml of anhydrous THF. The reaction medium was cooled to 0°C with an ice bath and 28ml (44.8mmol) of n-BuLi 1.6M in hexane were added dropwise. After 15 min. 4.2ml (54.0mmol) of DMF were added and the mixture was stirred at room temperature during 30 min. THF was removed under reduced pressure and the yellow-brown oily liquid was extracted with 3X75ml $CH_2Cl_2$. Organic phases were washed with 2X200ml of water and then dried over $MgSO_4$. Yellow oil was recovered after removal of the solvent under vacuum and was purified by flash column chromatography onto silica gel using a hexane/ethylacetate 90:10 mixture as the eluent. 5.6g (28.6mmol) of pure product were obtained (yield 80%).

**Example 3 -** Synthesis of 5-octylthiophene-2-carbaldehyde

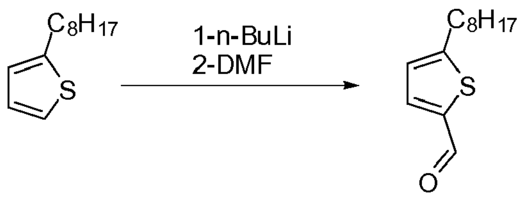


In a 250ml three necked round bottom flask were introduced under argon 8ml (36.0mmol) of 2-octylthiophene in 80ml of anhydrous THF. The reaction medium was cooled to 0°C with an ice bath and 28ml (44.8mmol) of n-BuLi 1.6M in hexane were added dropwise. After 15 min. 4.2ml (54.0mmol) of DMF were added and the mixture was stirred at room temperature during 30 min. THF was removed under reduced pressure and the yellow-brown oily liquid was extracted with 3X75ml $CH_2Cl_2$. Organic phases were washed with 2X200ml of water and then dried over $MgSO_4$. Yellow

oil was recovered after removal of the solvent under vacuum and was purified by flash column chromatography onto silica gel using a hexane/ethylacetate 90:10 mixture as the eluent. 5.4g (24.1mmol) of pure product were obtained (yield 67%).

**Example 4 -** Synthesis of 5-decylthiophene-2-carbaldehyde

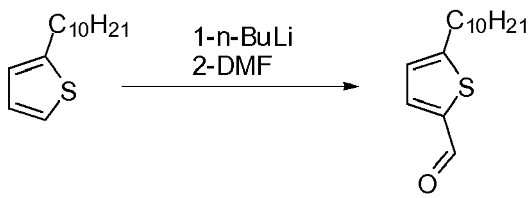


In a 250ml three necked round bottom flask were introduced under argon 10g (44.6mmol) of 2-decylthiophene in 80ml of anhydrous THF. The reaction medium was cooled to 0°C with an ice bath and 33ml (53.2mmol) of n-BuLi 1.6M in hexane were added dropwise. After 15 min. 5.0ml (64.3mmol) of DMF were added and the mixture was stirred at room temperature during 30 min. THF was removed under reduced pressure and the yellow-brown oily liquid was extracted with 3X75ml $CH_2Cl_2$. Organic phases were washed with 2X200ml of water and then dried over $MgSO_4$. Yellow oil was recovered after removal of the solvent under vacuum and was purified by flash column chromatography onto silica gel using a hexane/ethylacetate 90:10 mixture as the eluent. 9.0g (35.8mmol) of pure product were obtained (yield 77%).

**Example 6 -** Synthesis of 2-(thiophen-2-yl)decan-3-ol

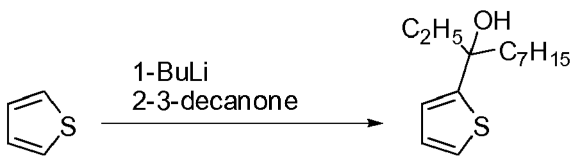


In a dried 250ml three necked round bottom flask were dissolved under argon 10g (60.3mmol) of thiophene in 60ml of anhydrous THF. The temperature was decreased down to -78°C and 42ml (67.2mmol) of n-BuLi 1.6M in hexane were added dropwise. Then, 11.6g (72.9mmol) of 3-decanone were added and the mixture was stirred during 2 hours -78°C and 16h at room temperature. Reaction was stopped by introducing small amount of water. Afterwards, solvent was removed under reduced pressure and the resulting crude product was purified by by flash column chromatography onto silica gel using a hexane/ethylacetate mixture as the eluent (95:5 to 90/10). 10.1g (41.9mmol) of a slightly yellow liquid product were obtained (yield 68.2%).

**Example 7** - Synthesis of 2-(decan-3-yl)thiophene

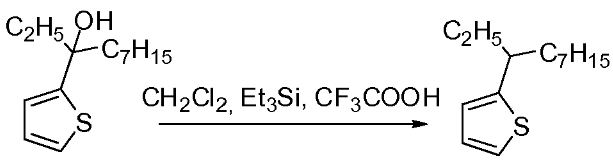


2-(decan-3-yl)thiophene

In a two necked round bottom flask were dissolved 4g (16.5mmol) of 2-(thiophen-2-yl)decan-3-ol in 150ml of $CH_2Cl_2$ and the medium was cooled to 0°C. Then, 5.3ml (32.9mmol) of triethylsilane and 1.5ml (19.4mmol) of trifluoroacetic acid were successively added dropwise. The reaction was stirred during 40min. at 0°C and then during 2 hours

at room temperature. Organic phase was successively washed with saturated sodium bicarbonate and saturated NaCl solutions before being dried over $MgSO_4$. After removal of the solvent under reduced pressure crude product was recovered and purified by flash column chromatography onto silica gel using hexane as the eluent. 3.03g (13.5mmol) of translucent liquid were obtained (yield 79.3%).

**Example 8 -** Synthesis of 5-(decan-3-yl)thiophene-2-carbaldehyde

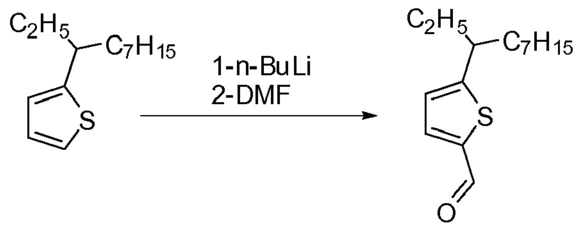


In a 100ml three necked round bottom flask were introduced under argon 2.78g (12.0mmol) of 2-(decan-3-yl)thiophene in 28ml of anhydrous THF. The reaction medium was cooled to 0°C with an ice bath and 10ml (16mmol) of n-BuLi 1.6M in hexane were added dropwise. After 15 min. 1.5ml (19.3mmol) of DMF were added and the mixture was stirred at room temperature during 30 min. THF was removed under reduced pressure and the yellow-brown oily liquid was extracted with 3X50ml of $CH_2Cl_2$. Organic phases were washed with 2X150ml of water and then dried over $MgSO_4$. Yellow oil was recovered after removal of the solvent under vacuum and was purified by flash column chromatography onto silica gel using a hexane/ethylacetate 90:10 mixture as the eluent. 2.74g (10.8mmol) of pure product were obtained (yield 87.6%).

**Example 9 -** Synthesis of (3Z,6Z)-3,6-bis((5-hexylthiophen-2-yl)methylene)piperazine-2,5-dione

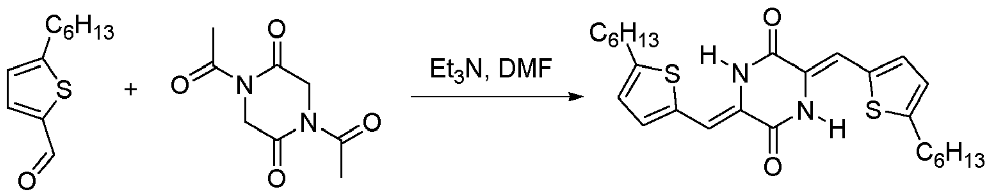


In a 50ml three necked round bottom flask were introduced under argon 4.04g (23.5mmol) of 5-hexylthiophene-2-carbaldehyde, 2.19g (10.6mmol) of 1,4-diacetylpiperazine-2,5-dione, 12ml (85.6mmol) of $Et_3N$ and 8ml of DMF. The reaction medium was heated at 90°C under stirring. A yellow solid was precipitated during its formation. After 16 hours the reaction mixture was cooled to room temperature and the solid was recovered by filtration and washed with 80ml of ethylacetate and 30ml of water. 3.14g (6.7mmol) of a bright yellow solid was obtained (yield 59.9%).

**Example 10 -** Synthesis of (3Z,6Z)-3,6-bis((5-octylthiophen-2-yl)methylene)piperazine-2,5-dione

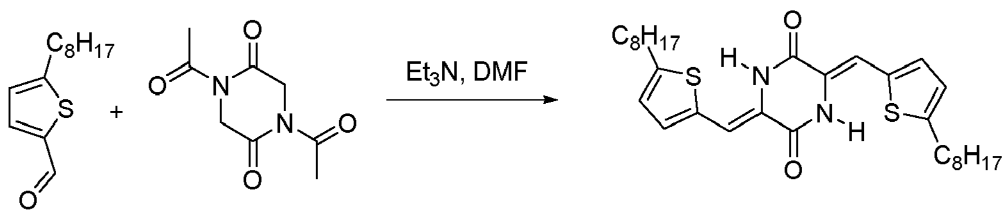


In a 50ml three necked round bottom flask were introduced under argon 7.5g (33.4mmol) of 5-octylthiophene-2-carbaldehyde, 2.9g (14.5mmol) of 1,4-diacetylpiperazine-2,5-dione, 17.5ml (125.4mmol) of $Et_3N$ and 11.5ml of DMF. The reaction medium was heated at 90°C under stirring. A yellow solid was precipitated during its formation. After 16 hours the reaction mixture was cooled to room temperature and the solid was recovered by filtration and washed with 100ml of ethylacetate and 100ml of water. 6.07g (11.5mmol) of a bright yellow solid was obtained (yield 75.5%).

**Example 11 -** Synthesis of (3Z,6Z)-3,6-bis((5-decylthiophen-2-yl)methylene)piperazine-2,5-dione

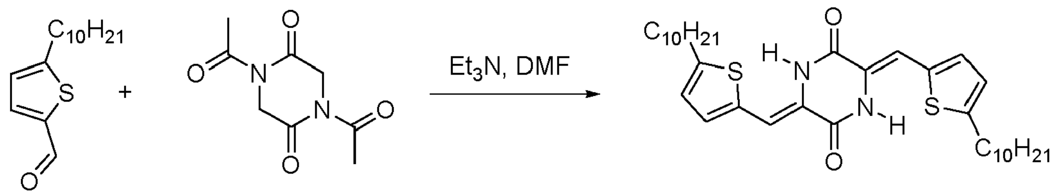


In a 50ml three necked round bottom flask were introduced under argon 8.5g (32.3mmol) of 5-decyllthiophene-2-carbaldehyde, 3.3g (16.5mmol) of 1,4-diacetylpiperazine-2,5-dione, 19ml (136mmol) of $Et_3N$ and 12ml of DMF. The reaction medium was heated at 90°C under stirring. A yellow solid was precipitated during its formation. After 16 hours the reaction mixture was cooled to room temperature and the solid was recovered by filtration and washed with 100ml of ethylacetate and 100ml of water. 5.27g (9.0mmol) of a bright yellow solid was obtained (yield 54.8%).

**Example 12 -** Synthesis of (3Z,6Z)-3,6-bis((5-(decan-3-yl)thiophen-2-yl)methylene)piperazine-2,5-dione

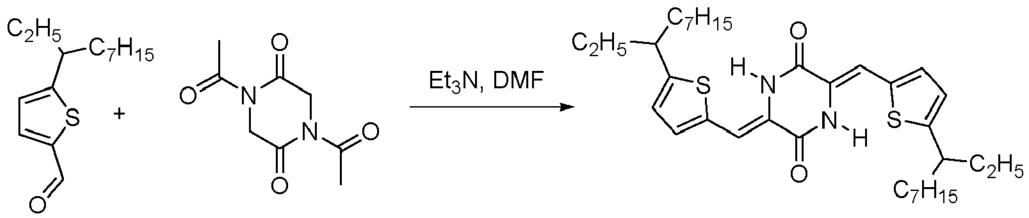


In a 25ml three necked round bottom flask were introduced under argon 2.5g (9.6mmol) of 5-(decan-3-yl)thiophene-2-carbaldehyde, 0.98g (4.9mmol) of 1,4-diacetylpiperazine-2,5-dione, 5.5ml (39.4mmol) of $Et_3N$ and 3.5ml of DMF. The reaction medium was heated at 90°C under stirring. After 16 hours the darkly colored reaction mixture was cooled to room temperature and crystallization occurred. The solid was recovered by filtration and washed with 100ml of ethylacetate and 100ml of water. 0.36g (0.6mmol) of a bright yellow solid was obtained (yield 12.5%). As this compound is more soluble in organic solvent than previously prepared compounds, some expected product may be recovered from evaporation of the filtrate.

**Example 13** - Synthesis of 1-(2-hexyldecyl)indoline-2,3-dione

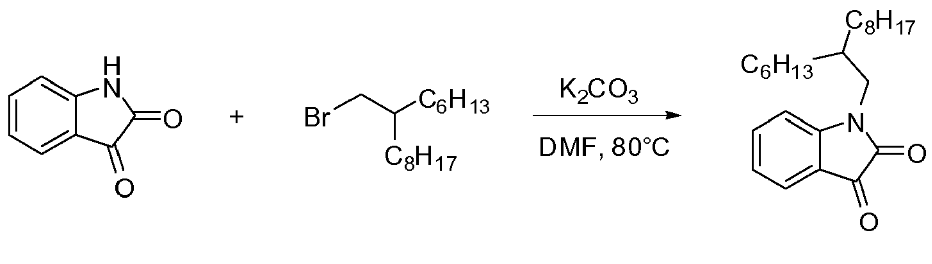


In a 1 l round bottom flask were dissolved 10g (68mmol) of isatin and 23.8g (78mmol) of 7-(bromomethyl)pentadecane in 500 ml of dry DMF. 37.6g (272mmol) of $K_2CO_3$ were introduced and the mixture was stirred at 80°C overnight.

Solvent was removed by distillation under reduced pressure and product was extracted with 400ml of ethyl acetate. Organic solution was washed with 2x200ml of water and 2x200ml of brine before being dried over $MgSO_4$. After evaporation of the solvent, 27 g of crude product was recovered and purified by flash column chromatography onto silica using $CH_2Cl_2$ followed by hexane/AcOEt (70/30) mixture as the eluent. 20.3g (55mmol) of pure product were obtained (yield 81.3%).

**Example 14** - Synthesis of 6-bromo-1-(2-hexyldecyl)indoline-2,3-dione

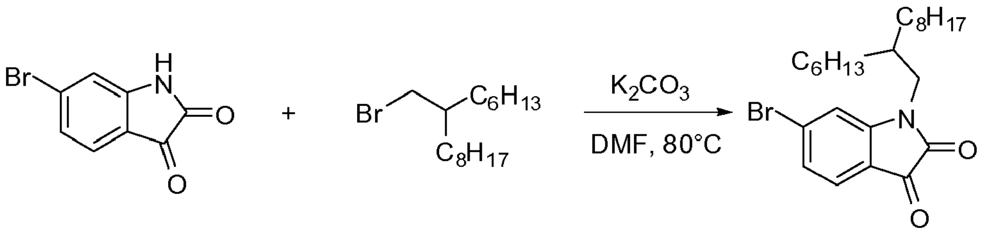


In a 1 l round bottom flask were dissolved 11.5g (50.9mmol) of 6-bromoisatin and 17.8g (58.3mmol) of 7-(bromomethyl)pentadecane in 300 ml of dry DMF. 29g (203mmol) of $K_2CO_3$ were introduced and the mixture was stirred at 80°C overnight. Solvent was removed by distillation under reduced pressure and product was extracted with 400ml of ethyl acetate. Organic solution was washed with 2x200ml of water and 2x200ml of brine before being dried over $MgSO_4$. After evaporation of the solvent, crude product was recovered and purified by flash column chromatography onto silica using hexane/AcOEt (70/30) mixture as the eluent. 9.2g (20.5mmol) of pure product were obtained (yield 40.3%).

**Example 15 -** Synthesis of (3Z,6Z)-3,6-bis(1-(2-hexyldecyl)-2-oxoindolin-3-ylidene)piperazine-2,5-dione

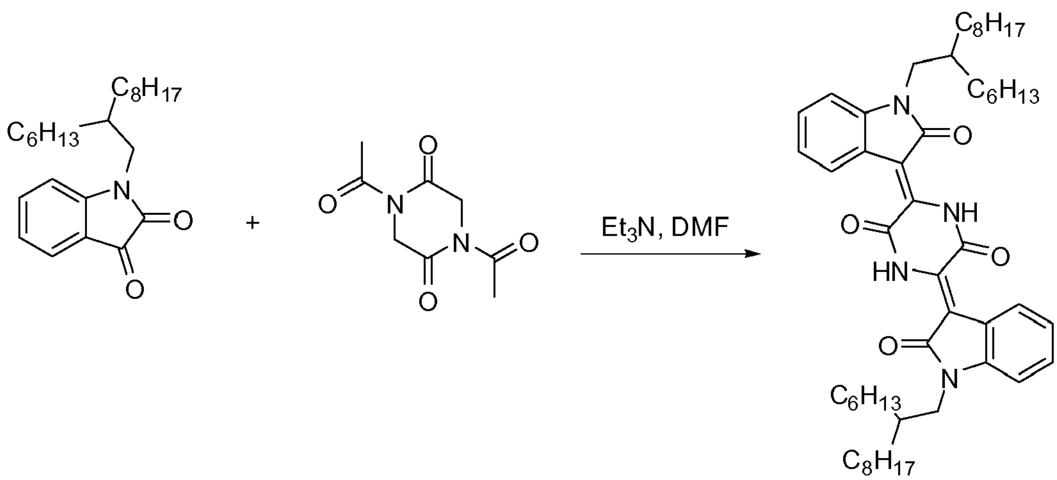


In a 100ml round bottom flask were dissolved 5g (13.5mmol) of 1-(2-hexyldecyl)indoline-2,3-dione and 1.33g (6.7mmol) of 1,4-diacetylpiperazine-2,5-dione in 50ml of dried DMF. Then, 1.5g (14.8mmol) of $Et_3N$ were introduced and the reaction mixture was stirred at room temperature for 5h. 4.8g (5.3mmol) of pure product were obtained by filtration followed by rinsing with MeOH (yield 79.1 %).

**Example 16 -** Synthesis of (3Z,6Z)-3,6-bis(6-bromo-1-(2-hexyldecyl)-2-oxoindolin-3-ylidene)piperazine-2,5-dione

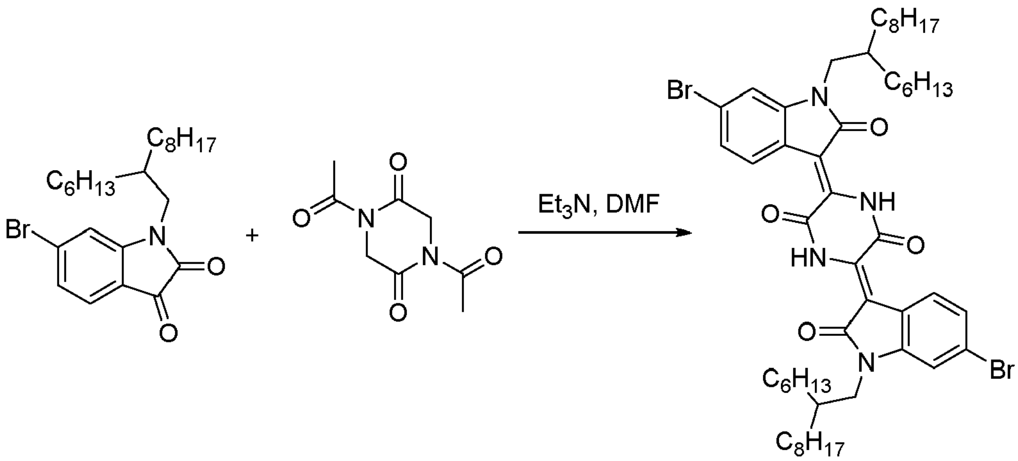


In a 100ml round bottom flask were dissolved 3.8g (8.4mmol) of 6-bromo-1-(2-hexyldecyl)indoline-2,3-dione and 0.84g (4.2mmol) of 1,4-diacetylpiperazine-2,5-dione in 15ml of dried DMF. Then, 0.95g (9.4mmol) of $Et_3N$ were introduced and the reaction mixture was stirred at room temperature for 24h. 3.8g (3.9mmol) of pure product were obtained by filtration followed by rinsing with MeOH (yield 92.9%).

The properties of the compounds synthesized in Examples 9, 12, 15 and 16 above are summarized in Table 1.

Table 1

| Ex. No. | OSC structure | LUMO (eV) | HOMO (eV) | Mobility ($cm^2$/Vs) |
|---|---|---|---|---|
| 9 | 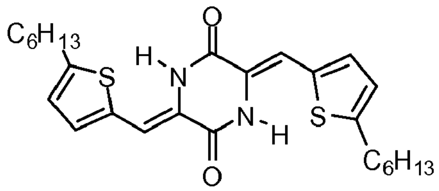 | -5.6 | -2.7 | - |
| 12 | 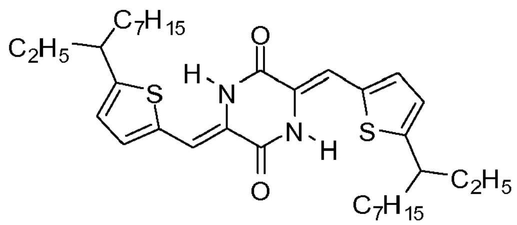 | -5.6 | -2.7 | $1.10^{-3}$ |
| 15 | 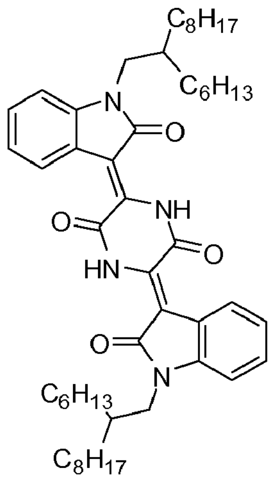 | <-5.8 | -3.6 | $1.10^{-4}$ |

(continued)

| Ex. No. | OSC structure | LUMO (eV) | HOMO (eV) | Mobility ($cm^2/Vs$) |
|---|---|---|---|---|
| 16 | 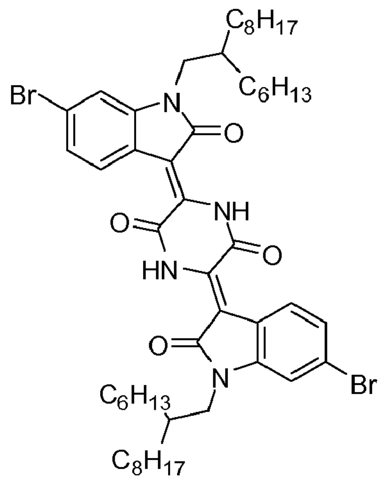 | <-5.8 | -3.7 | - |

The HOMO and LUMO levels of the organic molecules used in the process of the present invention were determined from cyclic voltammetry measurements in solution as follows:

The measurements are performed at room temperature, under inert atmosphere, with a conventional three-electrode configuration, the solution being outgassed before use with a stream of argon for 5 -10 min. The three-electrode cell may consist e.g of a glassy carbon disk as working electrode, a Pt wire or a Pt rod as a counter electrode and a Pt wire or a carbon rod as pseudo-reference electrode. Ferrocene is used as an internal reference. Other cell configurations may also be used. The solvents used for the determination of the HOMO and LUMO levels are respectively anhydrous dichloromethane and anhydrous tetrahydrofuran, the supporting electrolyte is 0.1 M tetrabutylammonium hexafluorophosphate and the host concentrations are 2 - 0.5 millimolar. The scan rate is fixed to 100 mv/s.

The HOMO levels ($E_{HOMO}$) of the organic molecules used in the process of the present invention are calculated from the measured half wave potential of their first oxidation wave ($E_{1\ ox\ 1/2}$) using the following equation:

$$E_{HOMO} - (-4.8) = -[E_{1\ ox\ 1/2} - E_{ox\ 1/2}(Fc/Fc^{+})]$$

wherein the ferrocene HOMO level value has been taken equal to -4.8 eV below the vacuum level according to Pommerehene and al. Adv. Mater. 7(6), 551-554 (1995) and wherein $E_{ox\ 1/2}(Fc/Fc^{+})$ corresponds to the measured half wave potential of the ferrocene oxidation wave. For irreversible systems, $E_{pa\ 1}$ peak potential value of the first oxidation wave is used instead of the half wave potential $E_{1ox\ 1/2}$.

The LUMO levels ($E_{LUMO}$) of the organic molecules used in the process of the present invention are calculated from the measured half wave potential of their first reduction wave ($E_{1ox\ 1/2}$) using the following equation:

$$E_{LUMO} - (-4.8) = -[E_{1\ red\ 1/2} - E_{ox\ 1/2}(Fc/Fc^{+})]$$

wherein the ferrocene HOMO level value has been taken equal to -4.8 eV below the vacuum level according to Pommerehene et al. Adv. Mater. 7(6), 551-554 (1995) and wherein $E_{ox\ 1/2}(Fc/Fc^{+})$ corresponds to the measured half wave potential of the ferrocene oxidation wave. For irreversible systems, $E_{pc\ 1}$ peak potential value of the first reduction wave is used instead of the half wave potential $E_{1\ red\ ½}$.

If no reduction wave is observed in tetrahydrofuran, the LUMO level is calculated from the HOMO level deduced from cyclic voltammetry as hereabove and from the optical energy bandgap $E_{opt\ g}$ measured from the wavelength of the absorption edge $\lambda_{abs.\ edge}$ using the following equations:

$$E_{opt\ g}\ (eV) = 1240/\lambda_{abs.\ edge}\ (nm); \quad E_{LUMO}\ (eV) = E_{HOMO}\ (eV) + E_{opt\ g}\ (eV).$$

If there is a risk of an overlap of the oxidation waves of ferrocene and the compound under evaluation, decamethylferrocene can be introduced into the medium as a reference. The equations used for the calculation of the HOMO and LUMO levels are then:

$$E_{HOMO} - (-4.8+( E_{ox\ 1/2}(Fc/Fc^{+})-E_{ox\ 1/2}(Me_{10}Fc/Me_{10}Fc^{+}))) = -[E_{1\ ox\ 1/2} - E_{ox\ 1/2}(Me_{10}Fc/Me_{10}Fc^{+})]$$

$$E_{LUMO} - (-4.8+( E_{ox\ 1/2}(Fc/Fc^{+})-E_{ox\ 1/2}(Me_{10}Fc/Me_{10}Fc^{+}))) = -[E_{1\ red\ 1/2} - E_{ox\ 1/2}(Me_{10}Fc/Me_{10}Fc^{+})]$$

where $E_{ox\ 1/2}(Fc/Fc^{+})-E_{ox\ 1/2}(Me_{10}FC/Me_{10}Fc^{+})$ are values reported in table 2 of D. Astruc et al., Can.J.Chem.84, 288-299 (2006).

For determining mobility as provided in Table 1, organic thin film transistor devices (OTFT's) with a Top Gate Bottom contact geometry were tested in air using a standard cascade probe station in conjunction with an Agilent B1501 semiconductor parameters analyzer. The Agilent system calculated the saturation mobility according to the equation

$$\mu_{sat} = \frac{2L}{W}\frac{1}{C_i}\left(\frac{\partial I_D^{0.5}}{\partial V_G}\right)^2_{V_D}$$

where L is the transistor length, W is the transistor width, $C_i$ is the dielectric capacitance per unit area, $I_D$ is the drain-source current and $V_G$ is the gate-source voltage. $V_D$ is the drain-source voltage and was set at - 100V.

The general structure of the devices used for measurement is shown in Fig. 1 wherein reference numeral 1 designates the Gate (Al), reference numeral 2 designates the dielectric layer, reference numerals 3 and 6 depict self assembled monolayers, reference numeral 4 stands for the drain (Au), reference numeral 5 stands for a buffer layer, reference numeral 7 depicts the source (Au), reference numeral 8 represents the organic semiconductor and reference numeral 9 depicts the bottom (glass).

## Claims

1. Use of compounds comprising at least one of structural elements (1) to (5) or of oligomers or polymers comprising units obtained from compounds of formulas (1) to (5)

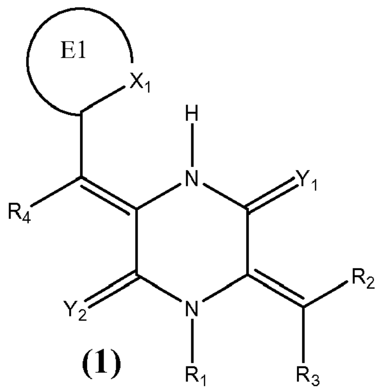

(1)

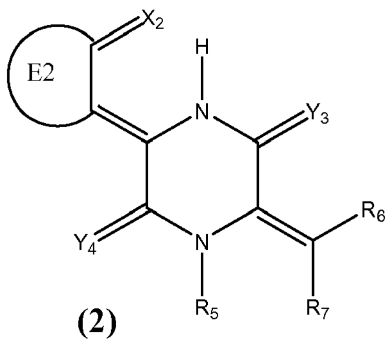

(2)

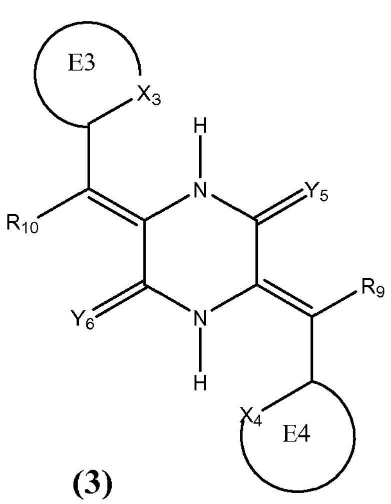

(3)

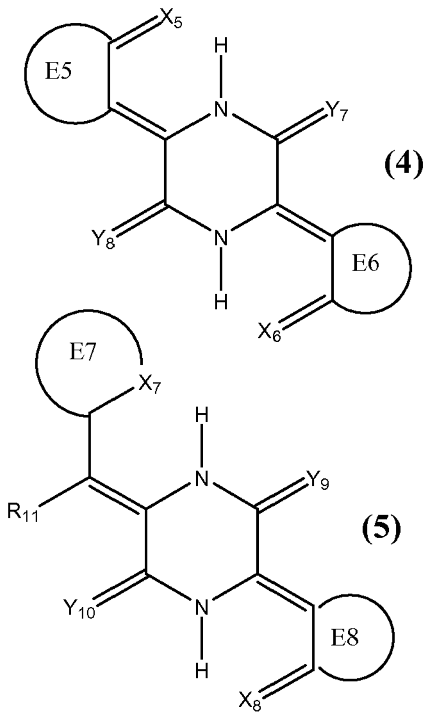

(4)

(5)

wherein

$X_1$, $X_3$, $X_4$ and $X_7$, which may be the same or different, represent hydrogen bond acceptors selected from the group consisting of -O-, -S-, -Se- and =N-, which hydrogen bond acceptors are part of rings E1, E3, E4 and E7, respectively

$X_2$, $X_5$, $X_6$ and $X_8$, which may be the same or different, represent hydrogen bond acceptors selected from the group consisting of O, S, Se and N-$R_{12}$, which hydrogen bond acceptors are attached to rings E2, E5, E6 and E8, respectively, via a double bond,

$Y_1$ to $Y_{10}$, which may be the same or different, represent O, S, Se or $NR_{13}$, E1 to E8, which may be the same or different, represent a 5- or 6-membered conjugated ring, which may be substituted or unsubstituted or fused with or bound to one or more additional conjugated rings, which may be substituted or unsubstituted, and

$R_1$ to $R_{13}$ represent hydrogen, a $C_1$-$C_{20}$- hydrocarbyl group or a $C_1$-$C_{20}$-heterohydrocarbyl group as organic semiconductors in organic electronic devices.

**2.** Use in accordance with claim 1 wherein the compounds, oligomers or polymers comprise structural elements of at least one of formulas (3) or (4) or units derived from at least one of formulas (3) and (4).

**3.** Use in accordance with claim 1 wherein the compounds, oligomers or polymers comprise structural elements of formula (6) or units obtained from formula (6)

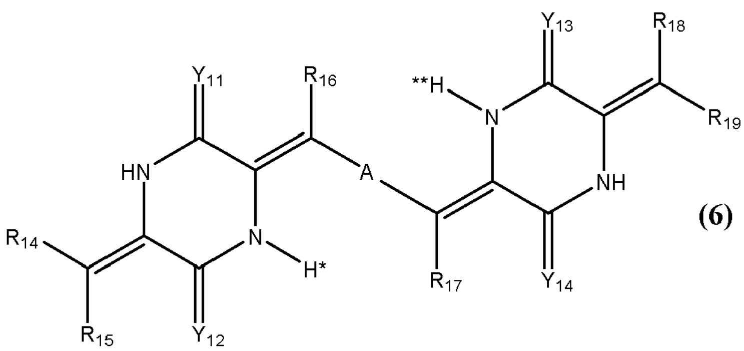

(6)

wherein $Y_{11}$ to $Y_{14}$, which may be the same or different, represent O, S Se or $NR_{20}$,

$R_{14}$ to $R_{20}$ represent hydrogen, a $C_1$-$C_{20}$ hydrocarbyl group or a $C_1$-$C_{20}$ heterohydrocarbyl group and

A represents a bidentate linking group selected from 6-membered heteroaryl rings or from systems comprising at least two connected or fused 5- or 6-membered conjugated rings, which may be substituted or unsubstituted, comprising two hydrogen bond acceptor groups selected from O, S. Se or N in the 6-membered heteroaryl ring or comprising at least one hydrogen bond acceptor group selected from O, S. Se or N in at least two of the connected or fused rings which form hydrogen bonds with the two hydrogen atoms H* and H**.

**4.** Use in accordance with claim 3 wherein A is selected from the group consisting of formulas (7) to (25)

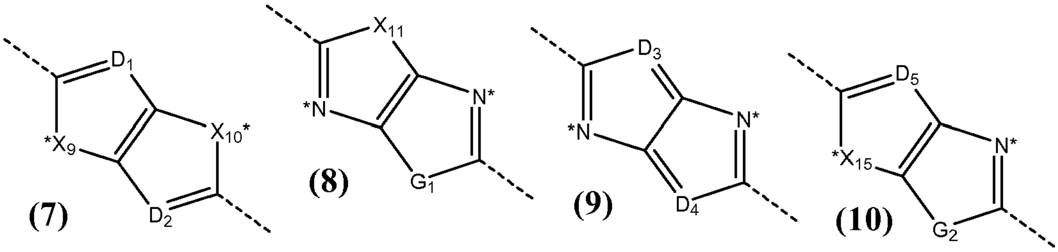

(7) (8) (9) (10)

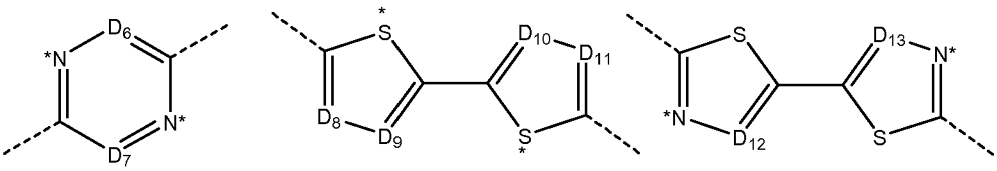

(11) (12) (13)

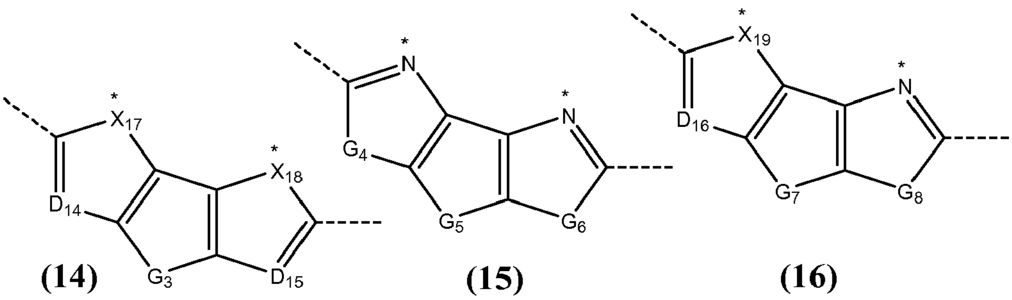

(14) (15) (16)

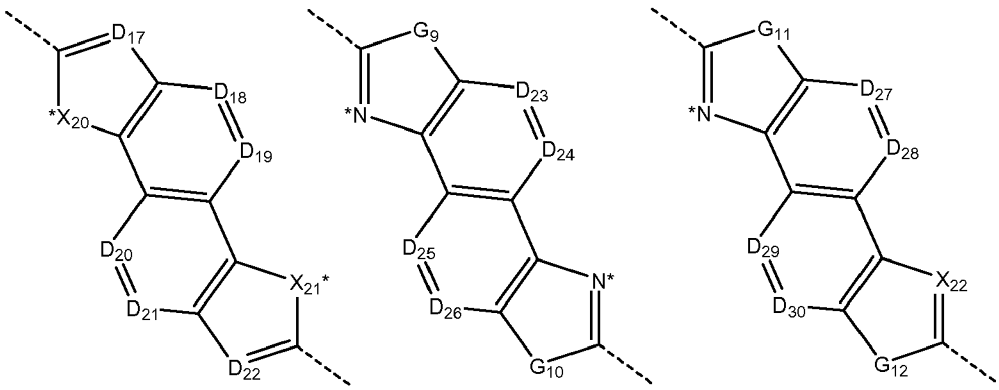

(17) (18) (19)

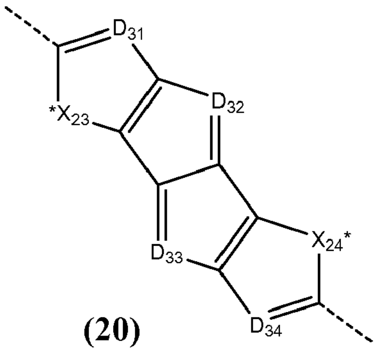

**(20)**

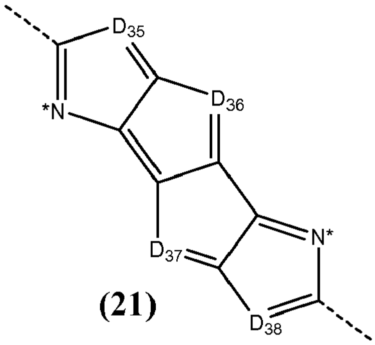

**(21)**

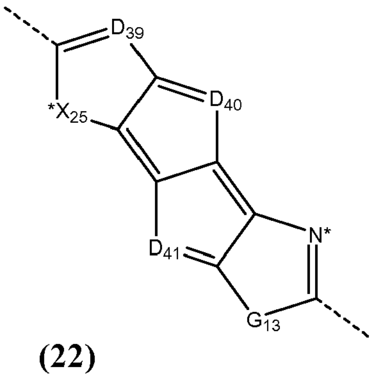

**(22)**

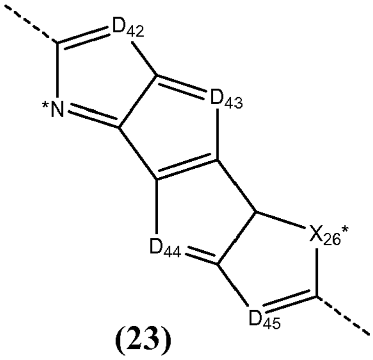

**(23)**

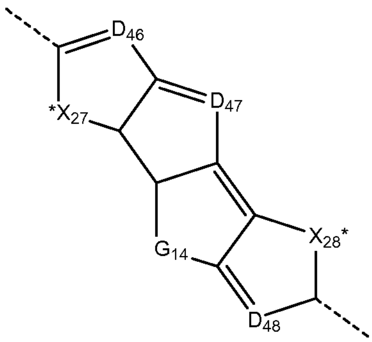

**(24)**

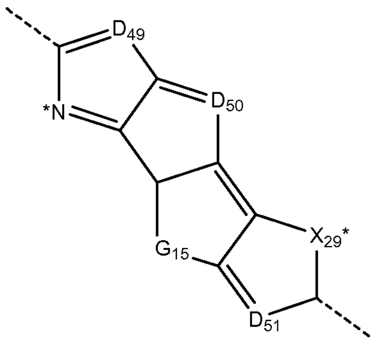

**(25)**

wherein
* denotes the hydrogen bond acceptor groups forming the hydrogen bond with H* and H** in formula (6),
the dashed line represents the linking atoms to the remainder of the molecule, $D_1$ to $D_{51}$, which may be the same or different, represent N or $CR_{21}$,
$G_{12}$ to $G_{15}$, which may be the same or different, represent O, S, Se, $NR_{22}$, C(=O) C(=S), $C(CR_{23}R_{24})$,
$R_{21}$ to $R_{25}$, which may be the same or different, represent hydrogen, a $C_1$-$C_{20}$ hydrocarbyl group or a $C_1$-$C_{20}$ heterohydrocarbyl group and
$X_9$ to $X_{29}$, which may be the same or different, represent a hydrogen bond acceptor group selected from -O-, -S-, -Se- or $-NR_{25}$.

**5.** Use in accordance with claim 3 wherein A is represented by formulas (26) to (28)

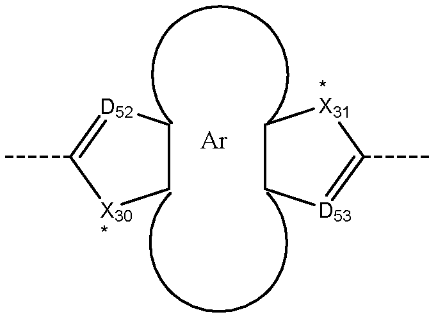

**(26)**

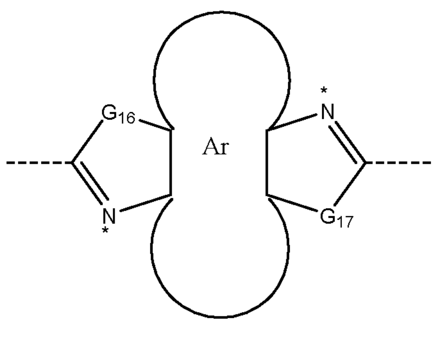

**(27)**

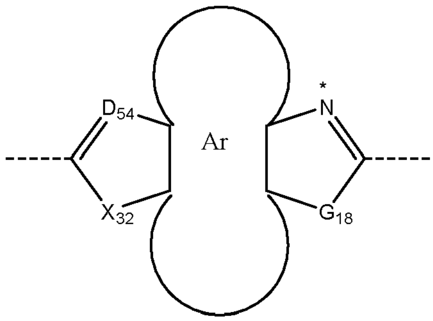

**(28)**

wherein

* denotes the hydrogen bond acceptor groups forming the hydrogen bond with H* and H** in formula (6),

the dashed line represents the linking atoms to the remainder of the molecule, $D_{52}$ to $D_{54}$, which may be the same or different, represent N or $CR_{26}$,

$G_{16}$ to $G_{18}$, which may be the same or different, represent O, S, Se, $NR_{27}$, C(=O) C(=S) or $C(CR_{28}R_{29})$,

$R_{26}$ to $R_{30}$, which may be the same or different, represent hydrogen, a $C_1$-$C_{20}$ hydrocarbyl group or a $C_1$-$C_{20}$ heterohydrocarbyl group and

$X_{30}$ to $X_{32}$, which may be the same or different, represent a hydrogen bond acceptor group selected from -O-, -S-, -Se- or $-NR_{30}$.

wherein Ar represents a six membered aromatic or heteroaromatic ring or an acene skeleton having two to 6 fused aromatic or heteroaromatic 6-membered rings which may be substituted or unsubstituted.

**6.** Use in accordance with claim 2 wherein the compound is represented by formulas (29) to (34)

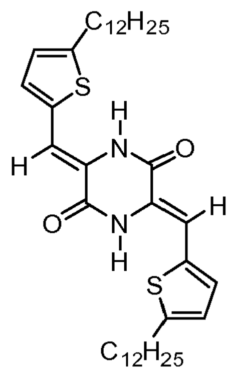

(29)

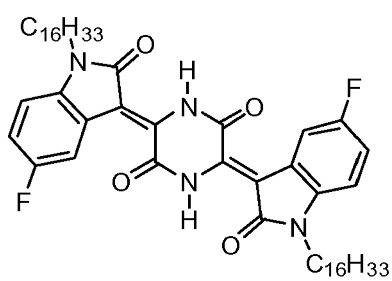

(30)

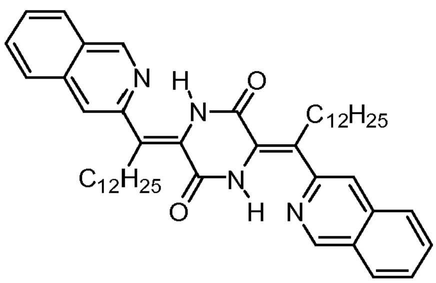

(31)

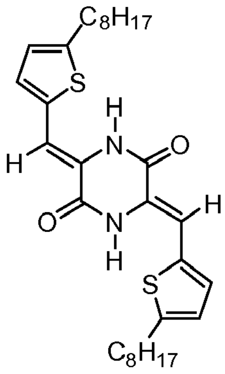

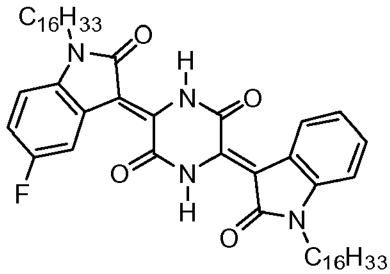

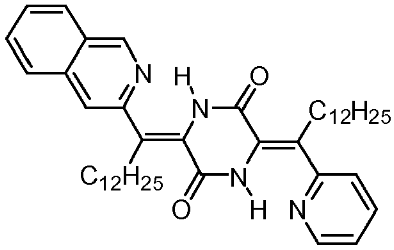

(32) (33) (34)

**7.** Use in accordance with claim 1 wherein the compounds are represented by formula (45) or the oligomers or polymers comprise repeating units of formula (46)

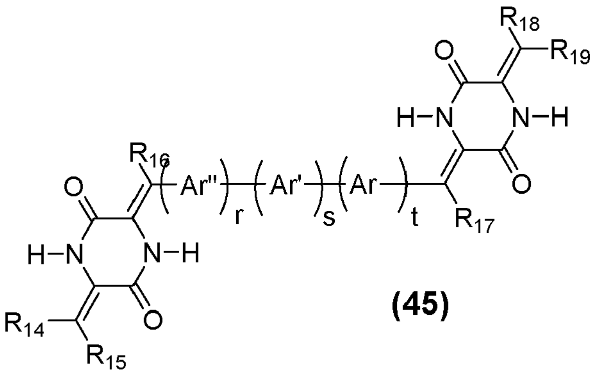

**(45)**

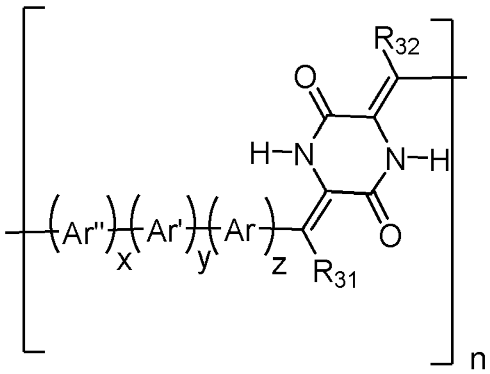

**(46)**

wherein

Ar and Ar'' represent 5- or six membered heteroaryl groups, which may be substituted or unsubstituted and which may form part of a fused ring system and which comprising at least one hydrogen bond acceptor group selected from O, S. Se or N which is capable of forming a hydrogen bond with the hydrogen atoms of the diketopiperazine group,

Ar' is an aryl or heteroaryl group with 1 to 30 carbon atoms, which may form part of a fused ring system,

$R_{14}$ to $R_{19}$ are as defined above,

$R_{31}$ and $R_{32}$, which may be the same or different, represent hydrogen, a $C_1$-$C_{20}$-hydrocarbyl group or a $C_1$-$C_{20}$-heterohydrocarbyl group, r,s and t, independent of one another, are an integer of from 0 to 5, the sum of r and t being at least 2,

x, y and z, independent of one another, are an integer of from 0 to 5, the sum of x and z being at least 2, and

n is an integer greater 1.

**8.** Compounds of formula (6)

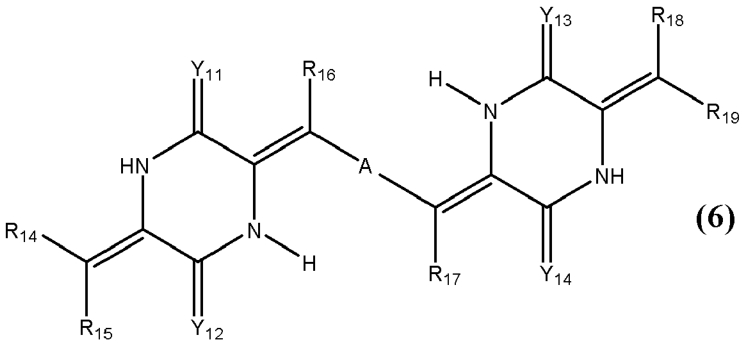

(6)

wherein $Y_{11}$ to $Y_{14}$, which may be the same or different, represent O, S Se or $NR_{20}$, $R_{14}$ to $R_{20}$ represent hydrogen, a $C_1$-$C_{20}$ hydrocarbyl group or a $C_1$-$C_{20}$ heterohydrocarbyl group and A represents a bidentate linking group selected from 6-membered heteroaryl rings or from systems comprising at least two connected or fused 5- or 6-membered conjugated rings, which may be substituted or unsubstituted, comprising two hydrogen bond acceptor groups selected from O, S. Se or N in the 6-membered heteroaryl ring or comprising at least one hydrogen bond acceptor group selected from O, S. Se or N in at least two of the connected or fused rings which form hydrogen bonds with the two hydrogen atoms H* and H**, or oligomers or polymers comprising repeating units obtained from compounds of formula 6.

**9.** Compounds or oligomers or polymers in accordance with claim 8 wherein A is selected from the group consisting of formulas (7) to (25)

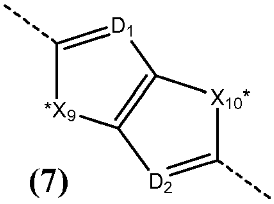

(7)

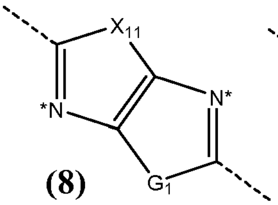

(8)

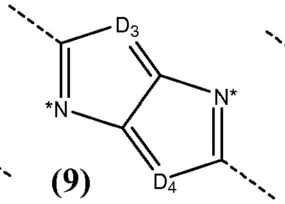

(9)

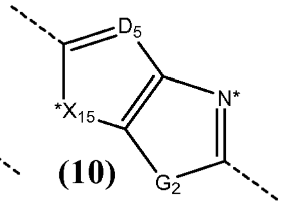

(10)

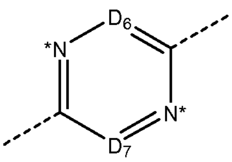

(11)

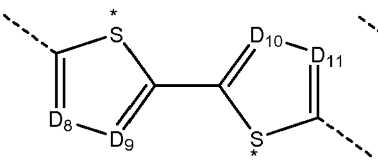

(12)

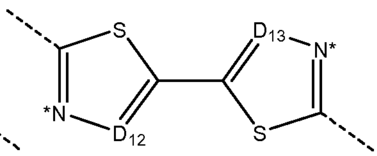

(13)

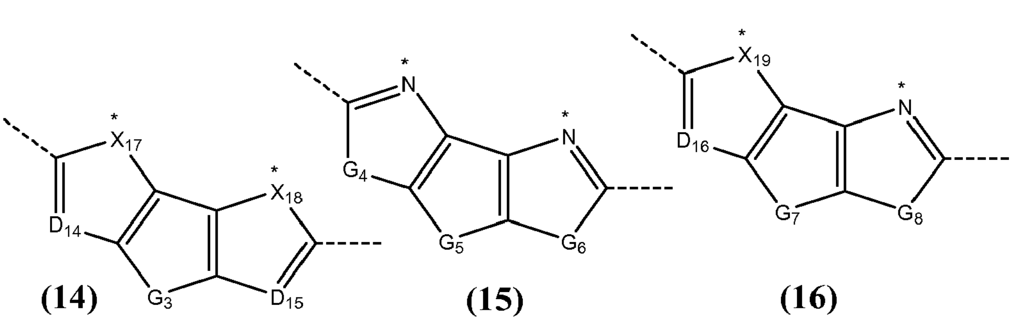

(14) (15) (16)

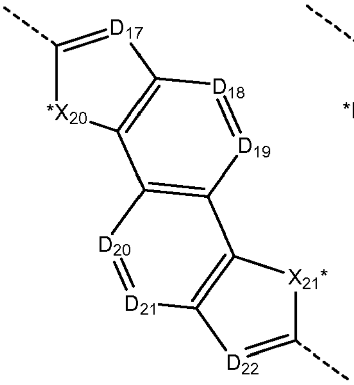

(17)

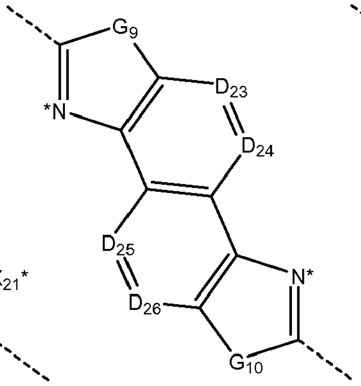

(18)

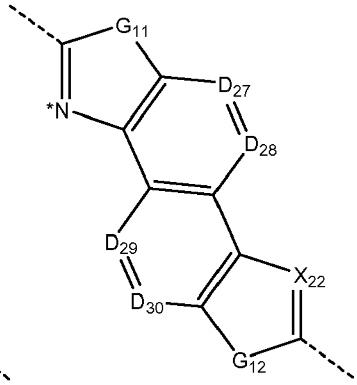

(19)

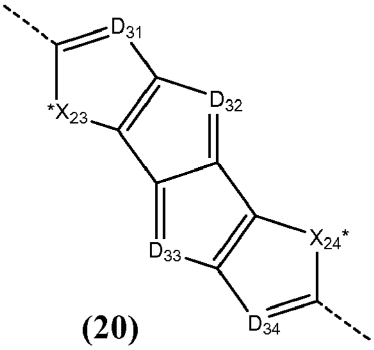

(20)

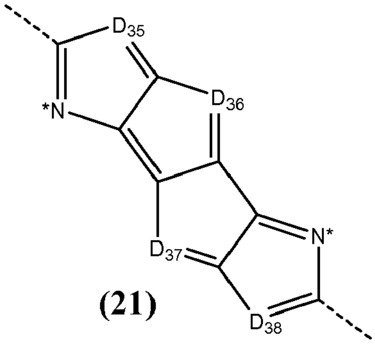

(21)

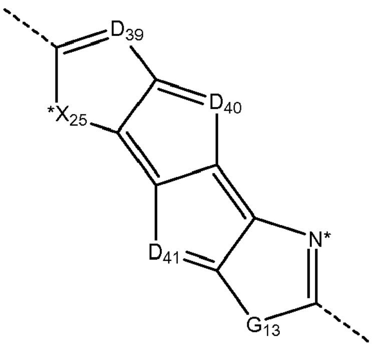

(22)

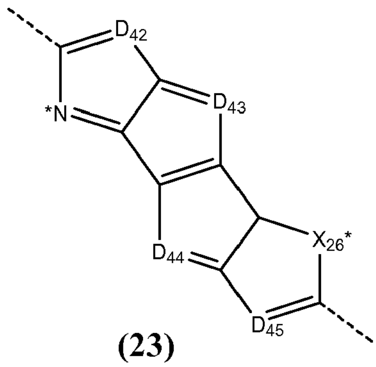

(23)

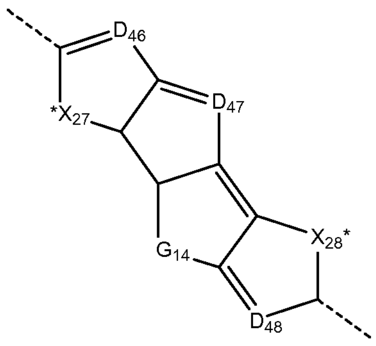

**(24)**

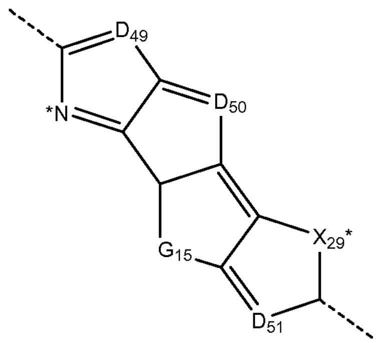

**(25)**

wherein
* denotes the hydrogen bond acceptor groups forming the hydrogen bond with H* and H** in formula (6),
the dashed line represents the linking atoms to the remainder of the molecule, $D_1$ to $D_{51}$, which may be the same or different, represent N or $CR_{21}$,
$G_{12}$ to $G_{15}$, which may be the same or different, represent O, S, Se, $NR_{22}$, C(=O) C(=S), $C(CR_{23}R_{24})$,
$R_{21}$ to $R_{25}$, which may be the same or different, represent hydrogen, a $C_1$-$C_{20}$ hydrocarbyl group or a $C_1$-$C_{20}$ heterohydrocarbyl group and
$X_9$ to $X_{29}$, which may be the same or different, represent a hydrogen bond acceptor group selected from -O-, -S-, -Se- or $-NR_{25}$.

**10.** Compounds or oligomers or polymers in accordance with claim 8 wherein A is selected from the group consisting of formulas (7) to (28).

**11.** Oligomers or polymers comprising repeating units of formula (46)

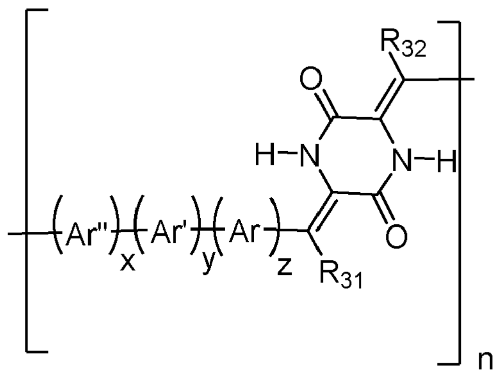

**(46)**

wherein
Ar and Ar'' represent 5- or six membered heteroaryl groups, which may be substituted or unsubstituted and which may form part of a ring system and which comprising at least one hydrogen bond acceptor group selected from O, S. Se or N which is capable of forming a hydrogen bond with the hydrogen atoms of the diketopiperazine group,
Ar' is an aryl or heteroaryl group with 1 to 30 carbon atoms, which may form part of a fused ring system,
$R_{31}$ and $R_{32}$, which may be the same or different, represent hydrogen, a $C_1$-$C_{20}$-hydrocarbyl group or a $C_1$-$C_{20}$-heterohydrocarbyl group, x, and z, independent of one another, is an integer of from 1 to 5,
z is an integer of from 0 to 5, and
n is an integer greater 1.

**12.** Organic electronic device comprising a compound comprising at least one of structural elements (1) to (6) or of oligomers or polymers comprising units obtained from compounds of formulas (1) to (6) and (43) or oligomers and

polymers of formula (46) as organic semiconductors.

**Figure 1**

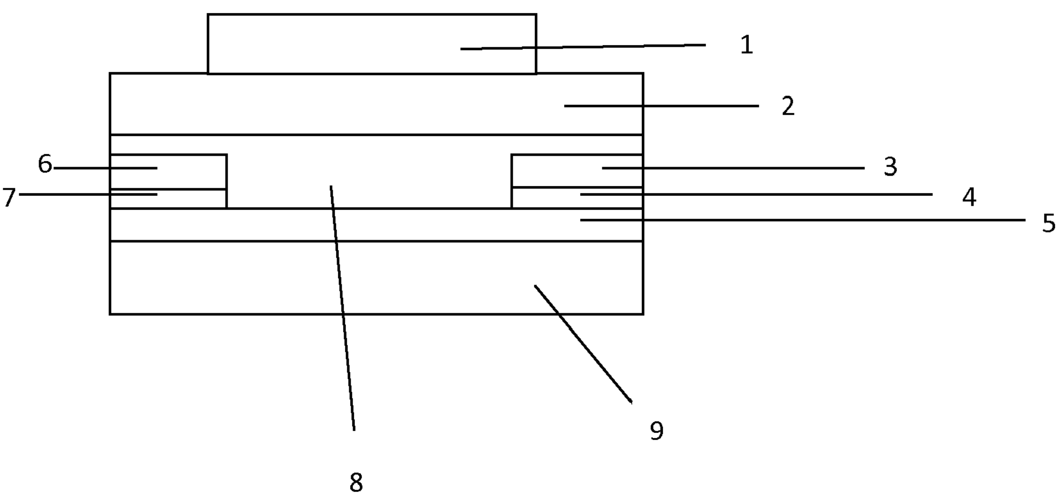

Europäisches Patentamt
European Patent Office
Office européen des brevets

# EUROPEAN SEARCH REPORT

Application Number
EP 13 19 8931

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | M. L. BOLOGNESI ET AL.: "Discovery of a Class of Diketopiperazines s Antiprion Compounds", CHEMMEDCHEM, vol. 5, 10 June 2010 (2010-06-10), pages 1324-1334, XP002724058, * the whole document * ----- | 1-12 | INV. H01L51/30 C07D241/02 C08G61/12 |
| A | KATRITZKY A R ET AL: "Conjugated systems derived from piperazine-2,5-dione", JOURNAL OF HETEROCYCLIC CHEMISTRY, WILEY-BLACKWELL PUBLISHING, INC, US, vol. 25, 1 March 1988 (1988-03-01), pages 591-597, XP002263044, ISSN: 0022-152X, DOI: 10.1002/JHET.5570250243 * the whole document * ----- | 1-12 | |
| A | WO 2012/035436 A1 (TOKYO UNIVERSITY OF PHARMACY AND LIFE SCIENCES [JP]; HAYASHI YOSHIO [J) 22 March 2012 (2012-03-22) * the whole document * ----- | 1-12 | TECHNICAL FIELDS SEARCHED (IPC): H01L C07D C08G |

The present search report has been drawn up for all claims

1

| Place of search | Date of completion of the search | Examiner |
|---|---|---|
| The Hague | 8 May 2014 | Königstein, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 8931

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2014

| Patent document cited in search report | Publication date | Patent family member(s) | Publication date |
|---|---|---|---|
| WO 2012035436 A1 | 22-03-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120232088 A

### Non-patent literature cited in the description


- **GOMPPER et al.** *Angew. Chem. Int. Ed.,* 1992, vol. 31 (9), 1202ff
- **KUNKEL et al.** *J. Med. Chem.,* 2008, vol. 51, 4563-4570
- **CASTER et al.** *J. Heterocycl. Chem.,* 1988, vol. 25, 591
- **PALENIK et al.** *J. Heterocycl. Chem.,* 1989, vol. 26, 821
- **MEIJER.** *Chem. Eur. J.,* 2000, vol. 6 (24), 4597-4603
- **VALIYAVEETTIL ; VETRICHETELVAN.** *Chem. Eur. J.,* 2005, vol. 11, 5889-5898
- **POMMEREHENE.** *Adv. Mater.,* 1995, vol. 7 (6), 551-554, Adv. Mater.
- **POMMEREHENE et al.** *Adv. Mater.,* 1995, vol. 7 (6), 551-554
- **D. ASTRUC et al.** *Can.J.Chem.,* 2006, vol. 84, 288-299